(19) Europäisches Patentamt
European Patent Office
Office européen des brevets

(11) **EP 1 107 789 B1**

(12) **EUROPEAN PATENT SPECIFICATION**

(45) Date of publication and mention
of the grant of the patent:
**25.06.2008 Bulletin 2008/26**

(51) Int Cl.:
*A61K 39/395* (2006.01)   *A61K 38/02* (2006.01)
*C07K 16/18* (2006.01)   *C12N 1/21* (2006.01)
*C12N 15/85* (2006.01)   *C12Q 1/68* (2006.01)
*A61P 37/06* (2006.01)

(21) Application number: **99946625.3**

(22) Date of filing: **24.08.1999**

(86) International application number:
**PCT/US1999/019337**

(87) International publication number:
**WO 2000/010603 (02.03.2000 Gazette 2000/09)**

(54) **COMPOSITIONS AND METHODS FOR PROTECTING ORGANS, TISSUE AND CELLS FROM IMMUNE SYSTEM-MEDIATED DAMAGE**

ZUSAMMENSETZUNGEN UND VERFAHREN ZUM SCHUTZ VON ORGANEN, GEWEBE UND ZELLEN VOR SCHÄDEN, DIE DURCH DAS IMMUNSYSTEM VERURSACHT WERDEN

COMPOSITIONS ET PROCEDES POUR PROTEGER DES ORGANES, TISSUS ET CELLULES CONTRE DES LESIONS INDUITES PAR LE SYSTEME IMMUNITAIRE

(84) Designated Contracting States:
**AT BE CH CY DE DK ES FI FR GB GR IE IT LI LU MC NL PT SE**

(30) Priority: **24.08.1998 US 97640 P**
**24.08.1999 US 382088 P**

(43) Date of publication of application:
**20.06.2001 Bulletin 2001/25**

(60) Divisional application:
**08000330.4**

(73) Proprietor: **THE BOARD OF TRUSTEES OF THE LELAND STANFORD JUNIOR UNIVERSITY**
**Palo Alto, CA 94304-1850 (US)**

(72) Inventors:
• **HOPE, Ernest, G.**
**Durham, NC 27707 (US)**
• **NEGRIN, Robert**
**Palo Alto, CA 94303 (US)**

(74) Representative: **Von Renesse, Dorothea**
**König Szynka Tilmann von Renesse**
**Patentanwälte Partnerschaft**
**Postfach 11 09 46**
**40509 Düsseldorf (DE)**

(56) References cited:
**EP-A1- 0 813 871**          **WO-A-95/21614**

• **HOPPE E-G ET AL: "HSP47, A HOUSEKEEPING SERINE PROTEASE INHIBITOR AND COLLAGEN CHAPERONE SHARES HOMOLOGY WITH THE ALPHA2-DOMAIN OF HLA-A2 AND INHIBITS NON-MHC I RESTRICTED T AND NK CYTOTOXICITY AGAINST VASCULAR ENDOTHELIAL CELLS VIA A NOVEL MECHANISM" BLOOD, W.B.SAUNDERS COMPAGNY, ORLANDO, FL, US, vol. 90, no. 10,SUPPLPART01, 15 November 1997 (1997-11-15), page 455A, XP009027960 ISSN: 0006-4971**
• **DATABASE WPI Section Ch, Week 199544 Derwent Publications Ltd., London, GB; Class B04, AN 1995-340205 XP002278849 & JP 07 233083 A (KUREHA CHEM IND CO LTD) 5 September 1995 (1995-09-05)**
• **SALOH ET AL.: 'Intracellular Interaction of Collagen Specific Stress Protein HSP47 with Newly Synthesized Procollagen' THE JOURNAL OF CELL BIOLOGY vol. 133, no. 2, 1996, pages 469 - 483, XP002926438**
• **VERRICO ET AL.: 'Expression of the Collagen-related heat shock protein HSP47 in fibroblasts treated with hyperthermia or photodynamic therapy' BRITISH JOURNAL OF CANCER vol. 76, no. 6, 1997, pages 719 - 724, XP002926439**

**Description**

## FIELD OF THE INVENTION

The field of the invention is compositions and methods for the protection of tissue, organs and cells from immune system mediated damage.

## BACKGROUND OF THE INVENTION

Bone marrow transplantation (BMT) represents one form of curative therapy for patients with malignant diseases. The beneficial effects of BMT are not only due to the high dose chemo- and radiation therapy but also to immune mechanisms termed the graft-versus-leukemia effect (GvL) (Sullivan et al., Blood 73:1720 (1989); Weiden et al., N. Engl. J. Med. 300:1068-1073 (1979)). Both natural killer (NK) cells and T-cell subsets contribute to GvL (Antin, Blood 82: 2273-2277 (1993); Hauch et al., Blood 75:2250-2262 (1990)). This is based on the observation that removal of T-lymphocytes from the stem cell product results in dramatically enhanced relapse rates following allogeneic BMT (Martin et al., Blood 66:664-672 (1985)). It has also been recently demonstrated that infusion of donor lymphocytes into patients, who have relapsed following T-cell depleted allogeneic BM transplant, results in a significant percentage of patients re-entering a complete remission (Collins et al., Clinical Oncology 15:433-444 (1997); Kolb et al., Blood 86:2041-2050 (1995); Porter et al., N. Engl. J. Med. 330:100-106 (1994)). The identification and *in-vitro* expansion of NK- and T- cell subsets with spontaneous or acquired GvL anti-tumor activity is an area of active investigation.

The understanding of biology of classical cytotoxic T lymphocytes (CTL) and their use of TCR $\alpha/\beta$, CD3 and CD8 in the context of appropriate MHC I expression on target cells is advanced (Guidos et al., J. of Exp. Med. 172: 835-845 (1990); Weissman, Cell 76:207-218 (1994)). However, the biology of the rarer subsets of cytotoxic T-cells and of NK cells which do not use the above molecules in the sense of classical MHC I restricted cytotoxicity remains a challenge. For example, the field of tumor immunology is challenged with the consistent finding of unorthodox T-cell cytotoxic activity and expansion in the context of auto and allo reactivity of tumor targets which lacks classical features of MHC I restriction (Hoglund et al., Immun. Rev. 155:11-28 (1997)). Several distinct T and NK cell populations have been expanded *in vitro* and utilized in-vivo as effector cells for adoptive immune therapy.

Lymphokine activated killer (LAK) cells are IL-2 dependent short term culture products with limited proliferative potential derived from NK cells. LAK cells recognize a broad array of tumor cell targets and autologous tumor cells *in-vitro*; however, their *in-vivo* efficacy is limited by the requirement for the co-application of IL-2 (Blaise et al., Eur. Cytokine Netw. 2:121-129 (1991); Fortis et al., Cancer Immunol. Immunother 33:128-132 (1991); Lee et al., J. Biol. Response Mod. 7:43-53 (1988); Nalesnik et al., Transplantation 63:1200-1205 (1997); Rosenberg, J. Biol. Response Mod. 3: 501-511 (1984); Rosenberg et al., New England J. of Med. 316:889-897 (1987); Rosenberg et al., J. of Exp. Med. 161: 1169-1188 (1985); Teichmann et al., Leuk. Res. 16:287-98 (1992). The systemic administration of IL-2 is associated with considerable toxicities which are characterized by endothelial destruction, both *in-vitro* and *in-vivo* (Siegel et al, J. of Clin. Oncology 9:694-704 (1991)). LAK cell therapy has led to significant morbidity and even fatalities associated with the vascular leak syndrome (VLS) (Glauser et al., Am. J. Med. Sci. 296:406-412 (1988); Kotasek et al., Cancer Res. 48: 5528-5532 (1988); Kozeny et al., J. Clin. Oncology 6:1170-1176 (1988); Rosenberg et al., Ann. Surg. 210:474-484.

Tumor infiltrating lymphocytes (TIL) require isolation from a surgical specimen. They usually have a high selectivity for their respective tumor. However, their generation tends to be cumbersome and associated with low yields. Specific tumor antigens are rarely available to ensure sufficient *in-vitro* expansion for either successive treatments, or dose escalations (Rosenberg et al., New. Engl. J. of Med. 319:1676-1680 (1988); Rosenberg et al., J. Nat. Canc. Inst. 86:1159-1166 (1994)).

Cytokine Induced Killer (CIK) cells are generated in the absence of target cells from peripheral blood mononuclear cells (PBMC) by *in-vitro* culture in presence of IFN-$\gamma$, OKT-3, and IL-2. CIK cells have superior *in-vivo* GvL activity as compared to LAK and are capable of purging SCID mice from lethal hematopoietic tumor burden (Lu et al. J. Immunol. 153:1687-1696 (1994); Schmidt-Wolf et al., Ann. Hematol. 74:51-56 (1997); Schmidt-Wolf et al., Exp. Hematol. 21:1673-1679 (1993)). Like other forms of activated NK or T cells, bulk cultured CIK show some undesired cytotoxicity against *in-vitro* cultured endothelium (EC).

Vascular leak syndrome (VLS) is a significant side effect associated with adoptive immunotherapy for treating diseases such as cancer. In particular, it has been reported that the use of IL-2 and the various forms of IL-2 activated NK and T-cells can lead to significant toxicity including VLS. However, no agent has been reported which is capable of inhibiting the lysis of endothelial cells by activated, non-classical MHC class I restricted NK- and T-cells (Blaheta et al., Immunology 94:213-220 (1998); Finnegan et al., Cancer 82:186-199 (1998); Utoguchi et al., Inflammation 21:223-233 (1997)).

Vascular damage by the immune system is one manifestation of an overall activation of the immune system. While activation of the immune response is extremely important to a host's health and proper functioning, there are a number of situations where such activation is undesirable. One particular area is associated with transplantation, where one rarely has an identical match between the donor and recipient of the MHC antigens. Another clinical situation is autoimmune disease. CTLs attack cells where the MHC and associated peptide are both endogenous, as occurs in diseases such as insulin-dependent diabetes mellitus (IDDM). Other undesirable immune system activation events are associated with such maladies as septic shock, rheumatoid arthritis, Crohn's disease, inflammatory bowel disease, asthma, graft versus host disease, coronary artery disease and other cardiomyopathies, adult respiratory distress syndrome, viral liver cirrhosis, and the like.

Immunosuppression has become a general approach in situations where the inhibition of activation of CTLs, NKs and other NK-like cells is desired. However, immunosuppressants such as corticosteroids, cyclosporine A, FK506, and the like, have numerous undesirable side effects including, for example, a general immunosuppressive effect on the host's entire immune system. There is, therefore, substantial interest in identifying new compositions which can specifically protect tissues from damage caused by cells such as T-lymphocytes and other NK-like cells, particularly CTLs, while having less of a universal immunosuppressive effect on the immune system and fewer side effects, so as to leave the host with a substantial proportion of the immune system for protection against adventitious infection. Methods and compositions for achieving such specific immunosuppression are herein provided.

## RELEVANT LITERATURE

Hoppe and Negrin, Blood, American Society of Hematology, Thirty-Ninth Annual Meeting, December 5-9 (1997), abstract no. 2019; Jiang et al., Immunology 87(3):481-486 (1996); Wada et al., Jpn. J. Cancer Res. 84(8):906-913 (1993): Krensky et al., Exp. Opin. Invest. Drugs 5:809 (1996); XP-00 227 8849 Kureha Chemicals, 1994 and JP 07233083 A (KUREHA CHEM IND CO LTD), 5 September 1995, abstract; WO 95/21614; Satoh et al., The Journal of Cell Biology, Vol. 133, No. 2, 469-483 (April 1996); Verrico et al., British Journal of Cancer 76(6):719-724 (1997) and EP 0 813 871.

The abstract Hoppe et. al. 1997 refers to the treatment of HUVEC with Brefeldin A which renders them resistant against activated CD3+ 16- 56+ CIK CTL and IL-2 activated NK cells. Furthermore, this abstract reports that the cloned recombinant HuHsp47 protects previously cytotoxicity sensitive HUVEC from non-MHC I restricted killing by CD3+56+ CIK CTL. The tested cells do not form part of the active immune system and allow no conclusion with respect to prevention or treatment of an immune-system mediated cell damage.

Satoh, 1996 deals with the location where Hsp47 associates and dissociates from the procollagen chains in Chick embryo fibroblasts and beyond this supports that the function of Hsp47 remains open (page 482, last paragraph). No pharmaceutical compositions for prevention or treatment of diseases related to immune-system mediated damage to cells, tissue or organs is reported in this document.

Verrico et al, 1996 deals with Hsp47 expression in murine skin fibroblasts after photodynamic injury and provides no indication of its possible function in the human immune system.

EP 0 813 871 deals with the inhibition of the Hsp47 production by Berberine derivatives in order to treat collagen overproduction diseases. It does not deal with the human immune system.

XP-00 227 8849 Kureha Chemicals, 1994 deals with the increase of collagen synthesis in rat osteoblasts and provide no basis for Hsp47 testing with respect to the human immune system.

WO 95/21614 published in 1995 provides compounds structurally related to Brefeldin but gives no indication of any use in upregulating Hsp47 to prevent immune system mediated damage.

## SUMMARY OF THE INVENTION

A pharmaceutical composition comprising HuHsp47 polypeptide, compositions and methods for protecting cells, organs and other tissues, such as vascular endothelial cells, from damage caused by lymphocytes, NK cells and NK-like cells are herein provided. These compositions find use either by themselves or in conjunction with other immunosuppressing agents or other therapeutic compounds as a combined therapy. The subject compositions may be contacted *ex vivo* with cells, tissue or an organ. In some embodiments, the then treated material is transplanted into a recipient. The subject compositions may also be introduced *in vivo* by any convenient means, in sufficient amount to substantially protect cells, organs and/or other tissues from immune system-mediated damage. The subject compositions are preferably HuHsp47-related immunoprotective polypeptides or nucleic acids encoding such polypeptides.

In a preferred embodiment, cells, tissue or organs are contacted with an immunoprotecting amount of an HuHsp47-related immunoprotective polypeptide. Examples of the diseased states which cause the immune mediated damage include various autoimmune disease, graft *vs*. host disease and host *vs*. graft disease.

The invention also includes chimeric molecules of Hsp47-related immunoprotective polypeptides and labelled forms thereof. Methods are also disclosed for adoptive immunotherapy wherein an expanded set of T lymphocytes is returned to a patient with an immunoprotecting amount of brefeldin or an HuHsp47-related immunoprotective polypeptide.

Methods are also disclosed for identifying cells which bind to Hsp47-related Immunoprotective polypeptides.

## BRIEF DESCRIPTION OF THE DRAWINGS

Figure 1 shows the nucleotide sequence and corresponding amino acid sequence of the human Hsp47 cDNA and protein, respectively.

Figure 2 discloses related sequences among Hsp47, HLA-A antigens and IL-12 as well as consensus sequences.

Figures 3A, 3B and 3C demonstrate CIK mediated lysis of tumor targets and endothelial cells (EC).

Figure 4 demonstrates CIK tumor lysis and EC lysis as a function of brefeldin A (BFA) concentration.

Figures 5A, 5B and 5C show immuno detection of induction of Hsp47 in untreated, $3\mu g/ml$ and $10\ \mu g/ml$ BFA treated endothelial cell extracts.

Figures 6A and 6B show FPLC purification of p46 from BFA-induced human umbilical vein endothelial cells (HUVEC) extracts.

Figure 7 shows the results from a HUVEC-CIK protection assay with purified, native Hsp47 (p46.5).

Figures 8A and 8B shows the cloning of a huHsp47 gene cassette via PCR directed introduction of cloning sites and electrophoretic separation of expression products.

Figure 9 shows that externally provided rHsp47 protects HUVEC cells from CIK-mediated lysis.

Figure 10 shows northern analysis that confirms the upregulation of Hsp47 mRNA by BFA treatment.

Figure 11 shows that recombinant expression of huHsp47 renders HUVEC cells resistant to CIK-mediated lysis.

Figure 12 shows that the peptide "AVLSAEQLR" which is shared by both Hsp47 and HLA-A2*201 protects HUVEC cells from CIK-mediated lysis.

Figure 13 demonstrates graft *vs*. host disease protection by recombinant Hsp47 in conjunction with bone marrow transplantation.

Figures 14A and 14B demonstrate the induction of Hsp47 and other proteins by BFA.

Figures 15A, 15B and 15C depict the structural domains of Hsp47, C-terminal truncation of various domains and the effect thereof on EC cell lysis.

## DETAILED DESCRIPTION OF THE INVENTION

Methods and composition are provided for protecting cells, organs and tissues, such as vascular endothelial cells, from immune system-mediated damage caused by activated lymphocytes, NK and other NK-like cells. The methods and compositions find use for the treatment of a variety of adverse indications that are mediated by activated immune system cells including, for example, septic shock, scleroderma, rheumatoid arthritis, Crohn's disease, inflammatory bowel disease, colitis, asthma, graft versus host disease, coronary artery disease and other cardio myopathies, adult respiratory distress syndrome, and viral liver cirrhosis, and the like.

The immunoprotecting compositions of the present invention comprise Hsp47-related immunoprotective polypeptides.

As used herein, the term "Hsp47-related immunoprotective polypeptides" refers to any polypeptide which has immunoprotecting properties which are similar to that of Hsp47 polypeptide as defined herein. For example, human Hsp47 polypeptide has been shown to protect endothelial cells from lysis from CIK cells. An Hsp47-related immunoprotective polypeptide would be one which shares either qualitatively or quantitatively this or other immunoprotective properties of human Hsp47.

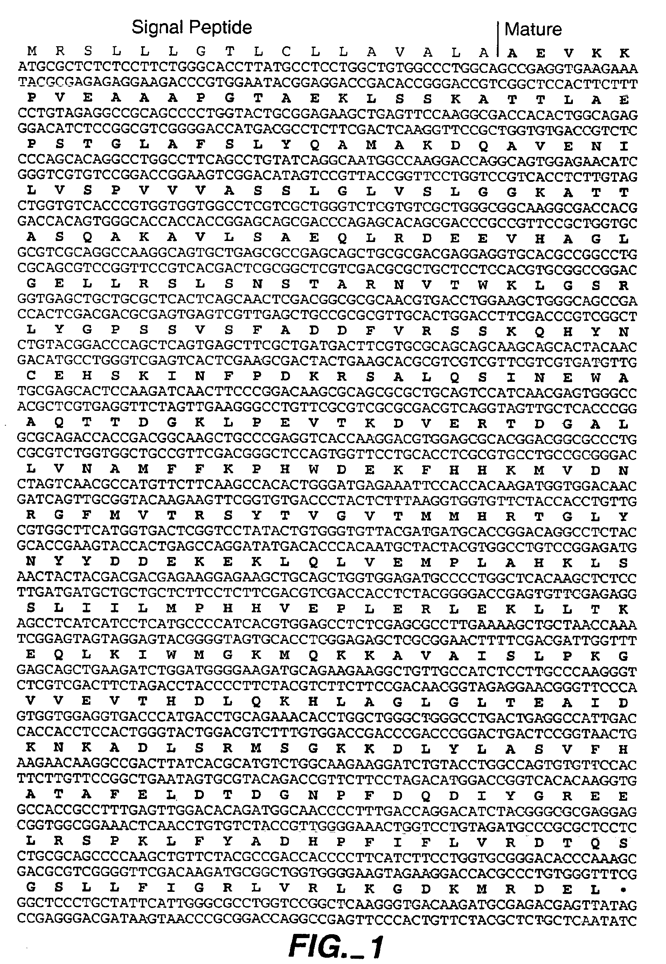

In one aspect of the invention, the Hsp47-related immunoprotective polypeptide additionally is characterized by an amino acid sequence motif which corresponds to the overall consensus sequence as set forth in Figure 1. This can be represented as $AX_1X_2X_3AX_4X_5X_6R$. In a preferred embodiment, $X_1$ is V, L, A or T, $X_2$ is L or H, $X_3$ is S or V, $X_4$ is D or E, $X_5$ is Q, K or R, and $X_6$ is L or V. This can alternatively be represented as A(v,l,a,t)(l,h)(s,v)A(d,e)(k,q,r)(l,v)R.

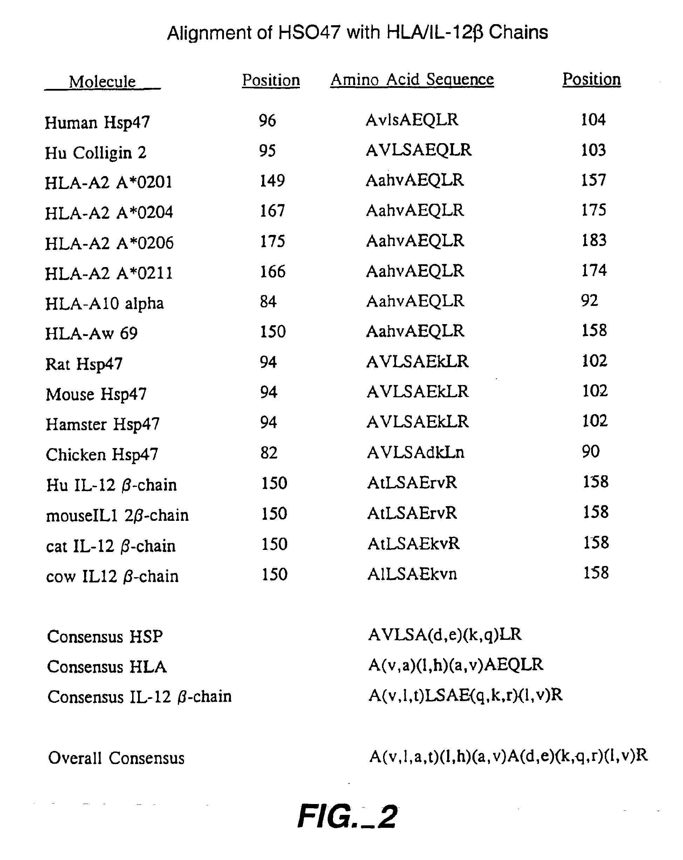

An Hsp47-related immunoprotective polypeptide can also comprise an IL-12 consensus sequence such as that shown in Figure 2. This can be represented as $AX_1LSAEX_5X_6R$ where $X_1$ is preferably V, L or T, $X_5$ is preferably Q, K or R, and $X_6$ is L or V. This aspect of the invention can also be represented as A(v,l,t)LSAE(q,k,r)(l,v)R.

In still a further aspect of the invention, the Hsp47-related immunoprotective polypeptide comprises the concensus sequence as set forth for HLA-A as set forth in Figure 1. This can be represented as $AX_1X_2X_3AEQLR$. In this embodiment, $X_1$ is preferably V or A, $X_2$ is preferably L or H, $X_3$ is preferably S or V. This aspect of the invention can also be represented as A(v,a)(1,h)(s,v)AEQLR.

In a particularly preferred aspect of the invention, the Hsp47-related polypeptide comprises the consensus sequence for Hsp as set forth in Figure 2. This can be represented as $AVLSAX_4X_5LR$. In this embodiment, $X_4$ is preferably D or E, and $X_5$ is preferably K or Q. This aspect of the invention can also be represented as AVLSA(d,e)(k,q)LR.

In still another preferred embodiment, the Hsp47-related immunoprotective polypeptide has the sequence $AX_1X_2X_3AEQLR$, wherein $X_1$, $X_2$ and $X_3$ can be any amino acid and Hsp47 polypeptides preferably comprising the sequence AVLSAEQLR.

Figure 2 shows the relationship between the peptide sequence spanning residues 96 through 104 of human Hsp47 as compared to similar motifs found in other specific HLA-A antigen molecules and IL-12. Accordingly, in the method of reducing immune mediated damage, any Hsp47-related immunoprotective polypeptide involving HLA-A, Hsp47 and IL-12 and immunoprotecting fragments or variants thereof.

As used herein, the term "Hsp47 polypeptide" refers to a polypeptide having the sequence set forth in Figure 1. The term also includes proteins which have at least 70% amino acid sequence identity with the sequence as set forth in Figure 1, more preferably greater than 90%, most preferably greater than 95% identity with the sequence set forth in Figure 1. The term also refers to a protein encoded by a nucleic acid which is capable of hybridizing with a nucleic acid having the sequence set forth in Figure 1 under moderate or stringent conditions as set forth in more detail hereinafter. The definition of Hsp47 polypeptide also includes immunoprotecting fragments and allelic variations as well as modifications introduced by recombinant technique to substitute, insert or delete one or more amino acid residues.

The entire human Hsp47 cDNA sequence is set forth in Figure 1A. It is within the skill level of the art to prepare purified recombinant or synthetic Hsp47 polypeptides. It is also well within the skill level of the art to identify other Hsp47 cDNA and polypeptides from vertebrates such as mammals and employ those non-human cDNAs and Hsp47 polypeptides in the subject compositions and methods. For example, counterparts to human Hsp47 are known in chicken and rat. As such, Hsp47 polypeptides as used herein include Hsp47 polypeptides and immunoprotecting fragments and variants thereof from all vertebrates, preferably mammals and most preferably humans. With regard to variants and fragments of full-length Hsp47 proteins that find use herein, "immunoprotecting" and grammatical equivalents thereof means that the variant(s) or fragment(s) are capable of reducing or eliminating damage to cells, organs or tissue that is caused by activated lymphocytes, NK cells or NK-like cells as compared to damage to the cells, organs or tissue that would be caused by such cells in the absence of the immunoprotecting agent.

With regard to peptide fragments of the full-length Hsp47 polypeptide or other Hsp47-related immunoprotective polypeptide that will find use herein, those amino acid sequence fragments will generally have at least about 8 consecutive amino acids, usually at least about 10 consecutive amino acids, preferably at least about 15 consecutive amino acids and more preferably at least about 20 consecutive amino acids of the Hsp47 protein sequence. The active sequence may be bonded or non-covalently linked within a chain or as a side chain of other peptides or proteins, for a variety of purposes.

The Hsp47 immunoprotective polypeptides as well as other Hsp47-related immunoprotective polypeptides that find use herein may comprise one or more amino acid substitutions, deletions or insertions. Typical conservative substitutions are shown in Table 1 under the heading of preferred substitutions. If such substitutions do not result in a change in biological activity, then more substantial changes, denominated exemplary substitutions in Table 1, or as further described below in reference to amino acid classes, are introduced and the products screened.

**TABLE 1**

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg leu; val; met; ala; phe; norleucine | arg |
| Ile (I) | leu; val; met; ala; phe; norleucine | leu |
| Leu(L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |

(continued)

| Original Residue | Exemplary Substitutions | Preferred Substitutions |
|---|---|---|
| Val (V) | ile; leu; met; phe; ala; norleucine | leu |

Hsp47 polypeptide-encoding nucleic acids that find use in the subject compositions and methods will have at least about 70%, usually at least about 75%, more usually at least about 80%, preferably at least about 85%, more preferably at least about 90% and most preferably at least about 95% nucleotide sequence identity with the corresponding nucleotide sequence shown in Figure 1A.

"Percent (%) amino acid sequence identity" with respect to the Hsp47 polypeptide sequences identified herein is defined as the percentage of amino acid residues in a candidate sequence that are identical with the amino acid residues in the wild-type human Hsp47 sequence (see Figure 1A), after aligning the sequences and introducing gaps, if necessary, to achieve the maximum percent sequence identity, and not considering any conservative substitutions as part of the sequence identity. "Percent (%) nucleotide sequence identity" with respect to the Hsp47 polypeptide-encoding sequences identified herein is defined as the percentage of nucleotides in a candidate sequence that are identical with the nucleotide sequence shown in Figure 1, after aligning the sequences and introducing gaps, if necessary, to achieve the maximum percent sequence identity. The % identity values used herein are generated by WU-BLAST-2 which was obtained from [Altschul et al., Methods in Enzymology, 266:460-480 (1996); http://blast.wustl/edu/blast/README.html]. WU-BLAST-2 uses several search parameters, most of which are set to the default values. The adjustable parameters are set with the following values: overlap span =1, overlap fraction = 0.125, word threshold (T) = 11. The HSP S and HSP S2 parameters are dynamic values and are established by the program itself depending upon the composition of the particular sequence and composition of the particular database against which the sequence of interest is being searched; however, the values may be adjusted to increase sensitivity. A % amino acid sequence identity value is determined by the number of matching identical residues divided by the total number of residues of the "longer" sequence in the aligned region. The "longer" sequence is the one having the most actual residues in the aligned region (gaps introduced by WU-Blast-2 to maximize the alignment score are ignored).

Hsp47 polypeptides or other Hsp47-related immunoprotective polypeptides that find use herein may be fused to heterologous amino acid sequences in order to provide chimeric or fusion proteins that will find use in the subject invention. Additionally, Hsp47 polypeptides and Hsp47-related polypeptides that find use herein may be obtained from recombinant expression of nucleotide sequences that encode the desired polypeptide(s). In this regard, nucleotide sequences that encode Hsp47 polypeptides of the present invention will hybridize to the nucleotide sequence shown in Figure 1 under moderately stringent conditions, preferably under stringent conditions. As used herein, "stringent conditions" means (1) employing low ionic strength and high temperature for washing, for example, 15 mM sodium chloride/ 1.5 mM sodium citrate (0.1x SSC)/0.1% sodium dodecyl sulfate at 50°C, or (2) employing during hybridization a denaturing agent, such as formamide, for example, 50% (vol/vol) formamide with 0.1% bovine serum albumin/0.1% Ficoll/0.1% polyvinylpyrrolidone/50 nM sodium phosphate buffer at pH 6.5 with 750 mM sodium chloride, 75 mM sodium citrate (5x SSC) at 42°C. Another example is use of 50% formamide, 5 x SSC (750 mM NaCl, 75 mM sodium citrate), 50 mM sodium phosphate (pH 6/8), 0.1% sodium pyrophosphate, 5 x Denhardt's solution, sonicated salmon sperm DNA (50 μg/ml), 0.1% SDS, and 10% dextran sulfate at 42°C, with washes at 42°C in 0.2 x SSC and 0.1% SDS. Yet another example is hybridization using a buffer of 10% dextran sulfate, 2 x SSC and 50% formamide at 55°C, followed by a high-stringency wash consisting of 0.1 x SSC containing EDTA at 55°C.

"Moderately stringent conditions" are described in Sambrook *et al., supra,* and include the use of a washing solution and hybridization conditions (*e.g.*, temperature, ionic strength, and %SDS) less stringent than described above. An example of moderately stringent conditions is a condition such as overnight incubation at 37°C in a solution comprising: 20% formamide, 5 x SSC (750 mM NaCl, 75 mM sodium citrate), 50 mM sodium phosphate (pH 7.6), 5 x Denhardt's solution, 10% dextran sulfate, and 20 mg/mL denatured sheared salmon sperm DNA, followed by washing the filters in 1 x SSC at about 37-50°C. The skilled artisan will recognize how to adjust the temperature, ionic strength, etc., as necessary to accommodate factors such as probe length and the like.

"Isolated," when used to describe the various Hsp47 polypeptides or Hsp47-related polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the Hsp47 polypeptide natural environment will not be present. Ordinarily, however, isolated polypeptide will be prepared by at least one purification step. Isolated Hsp47 polypeptides preferably do not contain Hsp47 specific antibodies bound thereto.

An "isolated" nucleic acid encoding Hsp47 polypeptide or Hsp47-related polypeptides is a nucleic acid molecule that is identified and separated from at least one contaminant nucleic acid molecule with which it is ordinarily associated in the natural source of the Hsp47-related polypeptide-encoding nucleic acid. An isolated Hsp47-related polypeptide-encoding nucleic acid molecule is other than in the form or setting in which it is found in nature. Isolated Hsp47-related polypeptide-encoding nucleic acid molecules therefore are distinguished from the Hsp47-related polypeptide-encoding nucleic acid molecule as it exists in natural cells. However, an isolated Hsp47-related polypeptide-encoding nucleic acid molecule includes Hsp47 polypeptide-encoding nucleic acid molecules contained in cells that ordinarily express Hsp47 polypeptide where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "antibody" is used in the broadest sense and specifically covers single anti-Hsp47 polypeptide monoclonal antibodies (including agonist, antagonist, and neutralizing antibodies) and anti-Hsp47 antibody compositions with polyepitopic specificity. The antibodies preferably do not bind to the epitopes reactive with MabN6 or Mab SPA470. In one aspect, the antibodies are specific for epitopes defined by the aforementioned consensus sequences. The term "monoclonal antibody" as used herein refers to an antibody obtained from a population of substantially homogeneous antibodies, *i.e.*, the individual antibodies comprising the population are identical except for possible naturally-occurring mutations that may be present in minor amounts.

The terms "treating", "treatment" and "therapy" as used herein refer to curative therapy, prophylactic therapy, and preventative therapy.

The term "mammal" as used herein refers to any animal classified as a mammal, including humans, cows, horses, dogs and cats. In a preferred embodiment of the invention, the mammal is a human.

Hsp47-related polypeptides or the fragments thereof may be labeled using standard techniques to incorporate radio labels, *e.g.*, $^{32}$P, fluorescent labels, etc. Additional moieties can be added to facilitate purification of the polypeptide either alone or in conjunction with binding to a cell. For example, Hsp47 polypeptides including a fluorescent label facilitates fluorescence activated cell sorting. Additionally, moieties which are capable of interacting with a magnetic field can be linked to Hsp47. In the latter case, magnetic beads attached to avidin can be used by combining them with biotin labeled Hsp47 polypeptide.

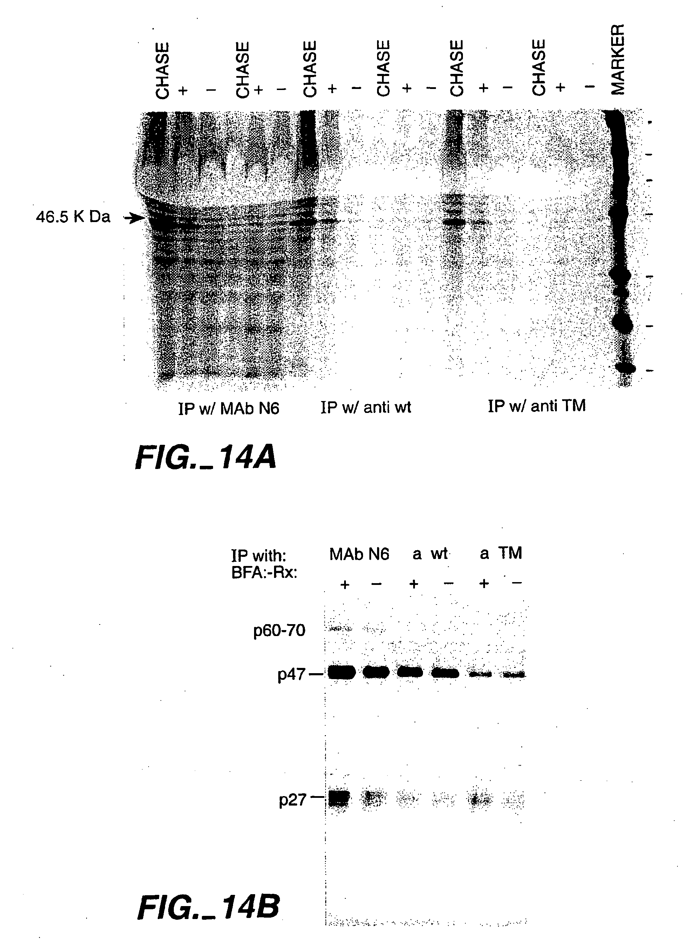

Hsp47 was identified as a protein induced in endothelial cells treated with brefeldin A. In addition to Hsp47 (identified as the 465 KDa band in Figure 14A and as p47 in Figure 14B), other proteins are induced by brefeldin A. These include the protein designated p27 in Figure 14B and a faint band above p47 in Figure 14B having a molecular weight of approximately 60-70 KDa. Such additional proteins may also be immunoprotective polypeptides useful in inhibiting the immune response alone or in combination with all the other proteins induced by brefeldin A.

The foregoing molecules can be used to identify cells that bind to the Hsp47 polypeptide or fragment thereof. Detecting the presence of such labels can be performed by any conventional technique, such as radiography, fluorescence detection, fluorescence activated cell sorting and divergence in a magnetic field depending upon the particular type of modified Hsp47 polypeptide used.

The subject compositions find use in a variety of ways. For research purposes, they may be used for analyzing the physiological pathway associated with immunoprotection and/or activation and inactivation of T lymphocytes, NK cells and other NK-like immune system cells as well as immunoprotection of various mammalian tissues, including vascular endothelium. For example, one can combine lymphocytes, particularly CTL cell lines having known peptide targets in conjunction with the subject compositions, in the presence and absence of antigen presenting cells to which the CTLs are restricted. After the lysis by the CTLs, one may then separate the activated CTL cells from quiescent CTL cells by means of the marker CD69, which marker is upregulated *in vitro* upon activation. Separation can be achieved using a FACS and a fluorescent labeled anti-CD69.

By isolating the most fluorescent cells, e.g. the highest 25%, one then lyses the cells and isolates proteins associated with the subject markers, e.g. chromatography, non-denaturing electrophoresis, or the like. Alternatively, one separates the proteins using electrophoresis and then uses a Western blot or other technique with the labeled peptides to identify proteins with which the subject peptides bind. Instead of a radiolabel, any other type of label may be employed, normally a small organic molecule, such as biotin, a fluorescer, and the like. Where biotin is used, after separation, avidin may be added, where the avidin is labeled with a label as described previously.

One may also compare lymphocytes which have been combined with antigen presenting cells in the presence and the absence of the subject immunoprotecting compositions. cDNA libraries may be prepared in each instance and representational differential analysis, subtraction, or the like may be employed to detect the differences in expression between the cells which have been activated in the presence and the absence of the subject compositions. One may also determine whether particular subsets of lymphocytes or other immune system cells respond differently from other subsets to the subject immunomodulating compositions by their expression or lack of expression or one or more proteins, particularly surface membrane proteins. In this way, lymphocytes may be identified which may be removed by leukophoresis or the like, in order to diminish an unwanted lymphocyte attack on tissue.

Depending upon their intended use, particularly for administration to mammalian hosts, the Hsp47-related immunoprotective polypeptides, *e.g.*, Hsp47 polypeptides, may be modified to change their distribution in the blood stream, diminish or enhance binding to blood components, enhancing the lifetime of the polypeptide in the blood stream, and the like. Immunoprotective polypeptides may be bound to these other components by linkers which are cleavable or non-cleavable in the physiological environment of the blood. Immunoprotective polypeptides may be joined at any point of the polypeptide where a functional group is present, such as hydroxyl, thiol, carboxyl, amino, or the like. Desirably, binding will be at either the N-terminus or the C-terminus.

The Hsp47-related immunoprotective compositions of the present invention may also be joined to a wide variety of other oligopeptides or proteins for a variety of purposes. For example, components of the subject compositions may be covalently linked to an immunogen to produce antibodies to components of the subject compositions, where the antibodies may serve for identification of other peptides having a comparable conformation. In addition, the antibodies may be used to prepare anti-idiotypic antibodies which may compete with the subject proteins or peptides for binding to a target site. These anti-idiotypic antibodies may then be used for identifying proteins to which the subject proteins and/or peptides bind. Alternatively, the immunoprotective polypeptides may be expressed in conjunction with other peptides or proteins, so as to be a portion of the chain, either internal, or at the N- or C- terminus. By providing for expression of the subject proteins and peptides, various post-expression modifications may be achieved. For example, by employing the appropriate coding sequences, one may provide for lipidation, *e.g.*, prenylation or myristoylation. In this situation, the immunoprotective polypeptides will be bound to a lipid group at a terminus, so as to be able to be bound to a lipid membrane, such as a liposome. For administration, liposomes may be used, where drugs may be introduced into the lumen of the liposome, so as to cooperate with the subject polypeptides in protecting tissues from immune system-mediated damage. Thus, immunosuppressants may be included in the lumen, so that the subject compositions and immunosuppressant may act in a localized manner.

Hsp47-related immunoprotective polypeptides such as Hsp47 may be PEGylated, where the polyethyleneoxy group provides for enhanced lifetime in the blood stream. The Hsp47-derived polypeptides or other immunoprotective polypeptides may also be combined with other proteins, such as the Fc of an IgG isotype, which may be complement binding or not bind complement, or with a toxin, such as ricin, abrin, diphtheria toxin, or the like, particularly the A chain.

The Hsp47-related immunoprotective polypeptides may be modified in a wide variety of ways. Sequence analogs may be prepared by oligopeptide synthesis using a stepwise substitution of the amino acids at each position with alanine or valine, particularly alanine. Generally the total number of amino acids substituted will not exceed 3, ranging from 1 to 3, usually 1 to 2. Methods of producing "scanning" mutations are known in the art, and have been successfully used with a number of different peptides. Examples of protocols for scanning mutations may be found in Gustin, et al. Biotechniques 14:22 (1993); Barany, Gene 37:111-123 (1985); Colicelli, et al. Mol Gen Genet 199:537-539 (1985), and Prentki, et al. Gene 29:303-313 (1984).

One can prepare these compositions by preparing a gene coding for an Hsp47-related immunoprotective polypeptide such as Hsp47 polypeptide (see, e.g., Figure 1A). The gene may be introduced into an appropriate expression vector, there being many expression vectors commercially available, whereby the gene is then expressed in an appropriate host. See, Sambrook et al., Molecular Biology: A Laboratory Manual, Second Edition, Cold Spring Harbor Laboratories, Cold Spring Harbor, NY, 1989.

The Hsp47-related immunoprotective polypeptides may be prepared by synthesis or by using recombinant techniques, as indicated above. Various commercial synthetic apparatuses are available, for example automated synthesizers by Applied Biosystems Inc., Foster City, CA, Beckman, etc. By using synthesizers, naturally occurring amino acids may be substituted with unnatural amino acids, particularly D-stereoisomers, side chains having different lengths or functionalities, and the like. For recombinant techniques, one may prepare a nucleic acid sequence which encodes a plurality of the subject polypeptides in tandem, with an intervening amino acid or sequence, which allows for cleavage to the single polypeptide or head to tail dimer. Where methionine is absent, one may have an intervening methionine which allows for single amino acid cleavage. Alternatively, one may introduce consensus sequences, which are recognized by particular proteases for enzymatic cleavage. The particular sequence and the manner of preparation will be determined by convenience, economics, purity required, and the like.

For the most part, the compositions which are used will comprise at least 20% by weight of the desired polypeptide product, more usually at least about 75% by weight, preferably at least about 95% by weight, and for therapeutic purposes, usually at least about 99.5% by weight, in relation to contaminants related to the method of preparation of the product and its purification. Usually, the percentages will be based upon total polypeptide.

By "brefeldin A" is meant the compound having the chemical formula 1,6,7,8,9,11a,12,13,14,14a-decahydro-1,13-dihydroxy-6-methyl-4H-cyclopent[f]oxacyclotridecin-4-one, which is commonly known in the art as brefeldin A, as well as any other members of the brefeldin A drug class or other drugs with the same or similar mechanism of action.

By "immunoprotecting amount" is meant the amount of brefeldin A or immunoprotecting polypeptide or expressible nucleic acid encoding it that is capable of reducing or eliminating the lymphocyte-, NK cell- or NK-like cell-mediated destruction of a tissue, preferably a vascular endothelial tissue. Immunoprotecting amounts may differ depending upon the indication for which the composition is employed and may be determined empirically and without undue experimentation by those of ordinary skill in the art.

With regard to brefeldin A, that drug, as well as drugs in the same class and drugs having the same or similar mechanisms of action, are well known and routinely employed in the art (see, e.g., Jiang et al., *supra* and Wada et al., *supra*).

The subject compositions including HuHsp47-related polypeptides and expressible nucleic acids encoding such immunoprotective polypeptides or combinations thereof can be used *in vitro* to inhibit lysis by T or NK cells of target antigen presenting cells, particularly vascular endothelial cells. Thus, in research where one wishes to maintain mixtures of cells, where lymphocytes would be activated and kill antigen presenting cells, such as macrophages or B-lymphocytes, or other cells which might serve as target cells, *e.g.*, neoplastic cells, viral infected cells, or the like, the lysis can be inhibited so that the cellular population may be maintained while under investigation.

The subject compositions including HuHsp47-related polypeptides and expressible nucleic acids encoding such immunoprotective polypeptides or combinations thereof can be used may also be used *ex vivo.* In cases of transplantation of organs or cells, more particularly solid organs or particular cells, *e.g.*, bone marrow, whether xenogeneic or allogeneic, the donor organ or cells may be bathed in a medium comprising the subject compositions. In this way, lymphocytes present with the implant will be inhibited from participating in graft versus host disease. Generally, the concentration of the composition will vary in the medium, depending upon the activity of the composition, the level of inhibition desired, the presence of other compounds affecting CTL activation, and the like. Usually, the concentration will be in the range of about 0.1 to 100 $\mu$g/ml of polypeptide, more usually in the range of about 1 to 10 $\mu$g/ml, although concentrations outside of these ranges may also find use. Other components of the bathing medium will generally be constituents normally used in an organ preservation solution, e.g. HBSS. The time for the organ to be maintained in the medium will generally be in the range of about 2 to 72 h.

The subject compositions including HuHsp47-related polypeptides and expressible nucleic acids encoding such immunoprotective polypeptides or combinations thereof may be also employed *in vivo,* administrating the subject compositions by any convenient means for the treatment of autoimmune diseases or to facilitate organ or cellular (*e.g.*, bone marrow) transplants. In the case of transplantation, the subject compositions may be administered prior to implantation, administration usually beginning not later than about 14 days prior to implantation, there preferably being at least one dosage administered within three days of administration. The subject compositions may be administered in the period beginning about 6 h prior to implantation and may be continued on a predetermined schedule thereafter, usually not past 30 days, more usually not past 20 days. However, after implantation, the subject compositions may be administered as needed, depending upon the response of the recipient to the organ or cells. In some situations, the subject compositions may be administered chronically, as long as the implant is present in the host. Other forms of *in vivo* use include injection of the subject compositions into areas of inflamation, *e.g.*, joints, ligaments, tendons and the like as well as various organs such as liver.

Other immunosuppressants which may be present during *in vitro, ex vivo* or *in vivo* treatment include injectable, parenteral or topical forms of cortisone, hydrocortisone, modified corticosteroids, Cyclosporin A, FK506, Imuran, azathioprine, D-pennicillamide, MMF (Mofetyl), Methotrexate, cyclophosphamide, gold preparations, salycilates, sufazalisine, antimalarials and NSAIDS. Biologic agents may also be used in conjunction with the subject compositions, including anti-CD5, Campath Mab, anti-CD4, diphtheria-IL-2 fusion toxin, soluble TNF-R and dimer, soluble IL-1-R,chimeric anti-TNF-alpha Mab, anti-ICAM-1 Mab, OKT3, anti-thymocyte serum/A6, anti-IL6, antiIL-2, antiCD7, as well as antibodies to CD4, CD8, CD3, LFA-1 and CD28. Subtherapeutic dosages will be employed, generally when present, not less than about 5% of the normal dosage, and not more than about 75%, usually in the range of about 10 to 60%.

In some instances, the subject compositions may be administered in combination with various immunostimulants. Such embodiments exist when the immune system has been compromised, for example, by way of chemotherapeutic agents. In such cses, it is desirable to boost the immune response. The use of the subject compositions in combination with immunostimulants such as IL-2 and other interleukins, interferons, cytokines or chemokines reduces the side effects associated therewith. Such side effects includ the amelioration of vascular leak syndrome which is often associated with treatments with immunostimulants.

Generally, a bolus of the subject composition which is administered will be in the range of about 0.1-50, more usually from about 1-25 mg/kg, of host. The host may be any mammal including domestic animals, pets, laboratory animals, primates, particularly humans. The amount will generally be adjusted depending upon the half life of the immunoprotecting drug or polypeptide, where the half life will generally be at least one minute, more usually at least about 10 min, desirably in the range of about 10 min to 12 h. Short half-lives are acceptable, so long as efficacy can be achieved with individual dosages or continuous infusion or repetitive dosages. Dosages in the lower portion of the range and even lower dosages may be employed, where the drug or polypeptide has an enhanced half life or is provided as a depot, such as a slow release composition comprising particles, introduced in a matrix which maintains the peptide over an extended period of time, e.g., a collagen matrix, use of a pump which continuously infuses the peptide over an extended period of time over a substantially continuous rate, or the like.

The transplantation may involve any organ or cells, including organs such as a heart, kidneys, lung, eyes, liver, gut, vascular vessel, or other organ, and cells, such as β-islet cells, bone marrow cells, or other cells, where the organ or cells are allogeneic or xenogeneic, particularly where one or more of the Class I or II MHC antigens are different in the donor as compared to the recipient.

The subject immunoprotecting compositions including HuHsp47-related immunoprotective polypeptides and nucleic acids encoding them, by themselves as conjugates or as combinations thereof, may be prepared as formulations in pharmaceutically acceptable media, for example, saline, PBS, aqueous ethanol, glucose, propylene glycol, or the like or as solid formulations in appropriate excipients, generally at a pharmacologically effective dose. The concentrations of the HuHsp47 polypeptide or other related immunoprotective polypeptide will be determined empirically in accordance with conventional procedures for the particular purpose. The formulations may include bactericidal agents, stabilizers, buffers, or the like. The amount administered to the host will vary depending upon what is being administered, the purpose of the administration, such as prophylaxis or therapy, the state of the host, the manner of administration, the number of administrations and the interval between administrations, and the like. In order to enhance the half life of the subject compositions, the compositions may be encapsulated, introduced into the lumen of liposomes, prepared as a colloid, or other conventional technique may be employed, which provides an extended life time of the compositions *ex vivo* or *in vivo*. In general, doses of immunoprotective amounts can be packaged in liquid or solid (*e.g.*, lypphilized) form in appropriate containers such as viles, *etc.* If liquid, the composition is preferably in a pharmaceutically acceptable medium (carrier). If solid, it should be prepared so that when reformulated as a liquid (*e.g.*, with water or a pharmaceutical carrier) a pharmaceutically acceptable composition is formed.

The examples disclose the effect of brefeldin A and Hsp47 polypeptides including the polypeptide AVLSAEQLR on CIK cell lysis of cultures of endothelial cells derived from human umbilical cord samples. These experiments demonstrated that CIK lysis of the endothelial cells is inhibited by brefeldin A and by at least Hsp47 which is expressed upon contacting the endothelial cells with brefeldin A. The following discussion summarizes the examples set forth herein as well as other experimental results. However, such results are merely exemplary of the scope of the invention in that the methods and compositions of the invention can be used to meliorate not only the undesirable lysis of endothelial cells but immune mediated damage to other cells, tissues and organs. Moreover, the effect of such compositions and methods is not limited to CIK cells but rather to non-MHC I restricted cytotoxic T lymphocytes and natural killer cells in general. In this regard, it should also be noted that while the methods and compositions of the invention inhibit non-MHC I restricted CTLs, other modes of immune response are not significantly affected. In this regard, a BMT experiment was performed on full body irradiated mice treated with or without an Hsp47 polypeptide. Graft vs. host disease appeared in the non-Hsp47 treated group. Of the members of the Hsp47 treated group, none developed graft *vs.* host disease. Moreover, none developed opportunistic infection over the time period tested which otherwise would have been expected if the entire immune system was suppressed.

The examples demonstrate that allogeneic and autologous CIK have a high cytotoxicity against a variety of hematopoietic and solid tumor targets including lymphoid, myeloid, and solid tumors as well as against *in-vitro* cultured EC. CIK also lyse additional cancer targets (Lopez et al., Faseb. J. 9:A1024 (1995); Lu et al., J. Immunol. 153:1687-1696 (1994); Mehta et al., Blood 86:3493-3499 (1995); Schmidt-Wolf et al., Ann. Hematol. 74:51-56 (1997)). All of the targets studied express normal levels of MHC I. Anti-MHC class I Mab W6/32, an antibody with proven capability to prevent or disrupt MHC I/TcR interactions *in-vitro* (Shields et al., Tissue Antigens 51:567-570 (1998)) does not block CIK mediated target lysis. The nature of the CIK mediated GvL effect was demonstrated to be a purely autologous process, in $^{51}$Cr release assays using cord-blood derived CIK and human umbilical cord derived endothelial targets from the same donor. This is consistent with the concept that CIK represent non classical MHC I restricted cytotoxic T-cells and excludes the possibility that this observed lysis represents the result of allo-recognition.

CIK are remarkable for their strong GvL effect without causing measurable GvHD in murine models, a finding also confirmed in man. This finding was documented with the SCID/hu *in-vivo* tumor purging model for B-cell lymphoma SU-DHL4 and chronic myelogenous leukemia (Lu et al., J. Immunol. 153:1687-1696 (1994); Hoyle et al., Blood 92: 3318-3327 (1998)). In the examples, a murine SCID system with orthotopic full-thickness human skin allografts was used to provide human cell adhesion molecules in the vessel walls of the skin grafts. This demonstrated lack of allorecognition and GvHD potential by CIK. The established skin grafts did not show signs of CIK infiltration, inflammation or GvHD. Only the human derived neoangiogenesis of solid tumors becomes the selective target of CIK mediated immune surveillance, but not physiologic normal vascular beds outside of the tumor vicinity.

Distinct differences in CIK target recognition become apparent in the analysis of cell adhesion molecules. Our studies demonstrate that the interaction of ICAM-1 on tumor targets with LFA-1 on the CIK effector cells is crucial for the process of CIK mediated lysis of tumor targets. This ICAM-1 / LFA-1 interaction appears not to be involved in CIK mediated EC lysis. These findings are consistent with previous reports on CD3$^+$56$^+$ T-lymphocyte requirements for target cell adhesion (Lu et al., J. Immunol. 153:1687-1696 (1994); Schmidt-Wolf, Cellular Immunology 169:85-90 (1996)) as opposed to their interaction with EC. CTLs utilize ICAM-1 and LFA-1 in their interaction with tumor targets. NK cells in the absence of MHC I/TcR ligation lyse targets which do not provide sufficient non-kill signalling, in situations where ICAM-1/LFA-1 provides the only identified stimulatory signal. The mechanism of T-cell co-stimulation through ICAM-1/LFA-1 ligation was recently discovered to involve myosin motor protein mediated accumulation of co-stimulatory signalling molecules in the T-cell/tumor target interface, leading to an amplified and prolonged signalling process (Wulfing et al., Science 282:2266-2269 (1998)). This process can be captured in real time via syn-capping micro-videography (Wulfing et al., Proc. Natl. Acad. Sci USA 95:6302-6307 (1998)). This ICAM-1/LFA-1 interaction is also crucial for the process of lymph node homing at high endothelial venules (Lawrence et al., Eur. J. Immunol. 25:1025-1031 (1995); Oppenheimer-Marks et al., J. of Immunology 145:140-148 (1990); Rosenman et al., J. of Leukocyte Biol. 53:1-10 (1993)). The ICAM-1/LFA-1 interaction seems to be not required for their interaction with non-activated EC, as this process was found to lack capping of LFA-1 and TcR in their interaction with EC (Kozeny et al., J. Clin. Oncol. 6:1170-1176 (1988)). The presence of a ICAM-1/LFA-1 dependent tumor lysis and an ICAM1/LFA-1 independent EC lysis process suggests the existence of at least two distinct pathways of CIK mediated cytotoxicity.

Single cell cloning of CIK demonstrated for the first time that endothelial lysis of activated non-classical MHC I restricted killer T-cells with anti-tumor activity is a clonal property of the activated T-cells. The mechanisms of cytotoxic endothelial and tumor recognition segregate independently from each other with individual CD3$^+$56$^+$ CIK clones expressing none, either one of the two, or both target recognition capacities. The vast majority of the CIK clones are $\alpha/\beta$ T-cells, but we also demonstrated that $\gamma/\delta$ T-cells can differentiate into cytotoxic CIK.

Lack of lytic ability by CIK of either the tumor or the EC target can be the result of at least three different clonal constellations: Each target class may require a specific cascade of signalling molecules to recognize it; lack of certain signalling cascade members in a particular CIK clone may lead to ineffective signalling for a given target. There may alternatively be a number of independently inhibitory signalling pathways interfering with the execution of a cytotoxic recognition event; protection from CIK lysis may result from a target cell's ability to express a respective ligand. Finally, sensitivity to a particular CIK killing mechanism may differ between tumor targets and EC; therefore individual clonal expression patterns and levels of alternate target attack mechanisms may account for the observed difference in effective target lysis. FACS analysis shows that targets express high levels of MHC I, and that functional CIK express $\alpha/\beta$ TcR (>95%) or $\gamma/\delta$ TcR (<5%), CD3/CD5 (>98%), CD8 (>80%) or CD4 (<15%), KIR molecules (5-8% staining for DX9) and CD56 (>25% in bulk cultures, >98% of functional clones) (Lu et al., J. Immunol. 153:1687-1696 (1994)). However, we have not been able to demonstrate functional involvement in target recognition and lysis by CIK for any cell adhesion molecules other than the above mentioned LFA-1 and ICAM-1 nor for the major T- and NK-surface proteins summarized in Table 2.

Effector cells were treated with CMA and BFA (brefeldin A) (Kataoka et al., J. Immunol. 156:3678-3686 (1996)) prior to $^{51}$Cr release cytotoxicity assay to estimate the contribution of perforin/granzyme versus Fas-based CIK cytotoxicity to these targets. Our results demonstrate that EC lysis is virtually entirely perforin and granzyme based, whereas tumor targets are killed utilizing both pathways. Previous research has demonstrated that CIK mediated lysis of tumor targets is cAMP and Ca2+ dependent (Mehta et al., Blood 86:3493-3499 (1995)).

The combined results from the FasL surface expression inhibition study with BFA and the perforin depletion study with Concanamycin A indicate that cytotoxicity against tumor targets is mediated via granzyme/perforin with significant contribution of the Fas/FasL pathway. EC lysis, in contrast, is mediated exclusively via granzyme/perforin. This result confirms that differences exist between EC and tumor target physiology which contribute to its sensitivity to CIK.

It is possible that CIK are anergic limited auto-reactive T-cells (Schwartz, Curr. Opin. Immunol. 9:351-357 (1997)). If so, they would represent a pool of T-cells with a heterogenic, differentiated CD complement, which may or may not have lost their function at that time point (Guidos et al., J. of Exp. Med. 172:835-845 (1990)). CIK culture like conditions might lead to rescue of anergic T-cells by providing high dose IL-2 stimulation and thus overcoming anergy (Madrenas et al., Proc. Natl. Acad. Sci. USA 93:9736-9741 (1996)).

Given the plurality of surface markers expressed on equally functional clones, the existence of a specific lineage of CIK T-cells and preferential expansion from a distinct precursor cell type found in peripheral blood appears unlikely. Rather, the presence of surface markers on T- and NK cells which lost their effective signalling capacity when tested functionally, has been reported as a hallmark characteristic of anergic cells (Fink et al., J. Immunol. 152:4270-4281 (1994)). Our understanding of the biology of T- and NK cells in the anergic state is still limited. But it has been implicated that high levels of IL-2 allow previously anergic T- and NK cells to resume cell division and to develop new, no longer auto-reactive, functional specificities (Alters et al., Transplantation 56:633-638 (1993); Hoglund et al., Immunological Reviews 155:11-28 (1997); Karpus et al., Int. Immunol. 6:721-730 (1994); Karre, Semin. Cancer Biol. 2:295-309 (1991); Ljunggren et al., Immunol. Today 11:237-244 (1990); Madrenas et al., Proc. Natl. Acad Sci. USA 93:9736-9741 (1996); Nossal, Ann. N. Y. Acad. Sci. 690:34-41 (1993); Rothenberg, Adv. Immunol. 51:85-214 (1992)). The high doses of IL-2 provided in CIK culture may thus rescue a heterogeneous pool of anergic PBMC T-cells which in the rescue process do not regain their prior potential for auto-reactivity. In addition to the de-novo expression of effector molecules involved in newly gained antitumor functions, CIK may therefore simply maintain their previous individual cell surface expression patterns of now non-functional receptors. This model allows for the observed heterogeneity of FACS markers present on equally functional CIK, as well as the loss of function associated with TcR/MHC I, CD4 and CD8 observed in our functional analysis.

Further insight into the process of cytotoxic T- and NK interaction with EC came from the finding that BFA specifically blocked EC lysis in a dose responsive fashion. This is the first report of an agent capable to suppress EC lysis by activated NK or T-cells. BFA induces a resistant EC state which persists for at least six hours after removal of the agent.

BFA treatment has been reported to lead to a complex series of changes in treated cells, via selective intoxication of a G-protein responsible for protein transport between cis- and medial golgi compartments. This transport block results in a backing-up of secretion directed proteins into an enlarging ER system and leads finally to the fusion of the cis-golgi compartments with the ER (Lippincott-Schwartz et al., Cell 56:801-813 (1989)). The enlargement of the ER compartment increases the synthesis of ER resident proteins. ER resident proteins are characterized by a C-terminal four amino acid "KDEL" or "RDEL"-ER retention signal. Examples of such ER-resident proteins are Hsp47, calreticulin, Grp78 and Grp94 (Ferreira et al., Arch. Virol. 138:273-285 (1994); Ferreira et al., J. Cell Biochem. 56:518-526 (1994); Ferreira et al., Connect Tissue Res. 33:265-273 (1996); Smith et al., J. Biol. Chem. 270:18323-18328 (1995)). These ER-resident proteins are not secreted out of the ER compartment, because they are in a $Ca^{2+}$ dependent fashion recognized, bound to and re-cycled back into the ER by the KDEL/RDEL-receptors, Erd2.1 and Erd2.2. The ER serves as major intracellular $Ca^{2+}$ store. Enlargement of the ER by BFA treatment perturbs the cellular $Ca^{2+}$ homeostasis and leads to inactivation of the KDEL/RDEL receptor (Llewellyn et al., Biochemical and Biophysical Research Communications 240:36-40 (1997)). ER resident proteins, including Hsp47, thus become freely secreted (Hu et al., J. Cell Biochem. 59:350-367 (1995); Hu et al., J. Cell Biochem. 59:214-234 (1995)). BFA treatment of EC therefore leads to an increased production of Hsp47 and to the free secretion of this otherwise ER resident protein. Under physiologic conditions, the transport of Hsp47 to the outside of the cell is linked to the expression and co-transport of chaperoned collagens I and IV (Yamamura et al., Biochem Biophys. Res. Comm. 244:68-74 (1998)).

$^{35}S^*$ pulse-chase labelled protein extracts of the BFA induced resistant state of EC were compared with the untreated sensitive phenotype. Amongst the consistently radio-labelled proteins increased in extracts from BFA treated EC was a protein of 46.5 KDa, p46.5. Using phase partitioning at 0-4°C with Triton-X-114 we were able to demonstrate p46.5 to be at least partially localized to the cell membrane of treated EC. Biotinoylation of the surface proteins of intact BFA treated EC monolayers with the water soluble, lipid insoluble reagent biotin-SS-NHS also demonskated Hsp47 and a 25-27 KDa associated protein to be upregulated at the cell surface after BFA treatment. See Figures 14A and 14B. Biochemically, p46.5 bound to and could be purified with gelatin-sepharose, eluting in both, competitive RGDpeptide binding and a low pH elusion buffers during FPLC preparations. All biochemical characteristics of p46.5 matched data published for rodent Hsp47 (Hirayoshi et al., Mol. Cell Biol. 11:4036-4044 (1991); Jain et al., Biochem J. 304:61-68 (1994); Nandan et al., Biochem Cell Biol. 68:1057-1061 (1990); Vaillancourt et al., Biochem J. 274:793-798 (1991)). Functional analysis demonstrated that gelatin purified p46.5 shares with BFA treatment the unique ability to protect previously CIK sensitive EC in a dose responsive fashion. This was shown through pre-incubation of EC with p46.5 prior to $^{51}Cr$ cytotoxicity assay. This is the first report of an externally added protein agent which protects EC from non-MHC I restricted cytotoxicity. This p46.5 protein cross-reacted in immuno-precipitations with antisera raised against membrane proteins from the resistant state of lymphoblastoid cells, and with purified pan-anti heat shock protein monoclonal antibody N6.

p46.5 was positively identified to be human Hsp47 on the original membrane of the N6 immunoprecipitation via the specific anti-Hsp47 monoclonal antibody SPA-470.

The full sequence of human *hsp*47 was first described by Clarke and Sandwal after PCR based isolation of partial, but overlapping, cDNA gene fragments spanning the entire human *hsp*47 gene (Clarke et al., Biochem Biophys Acta 1129:246-248 (1992)). Two plasmids were provided by Dr. Sanwal, corresponding to overlapping partial *hsp*47 cDNA fragments of 350 and 1300 bp, respectively. The smaller fragment also contained 5'untranslated regions not coding for the mature gene product of the *hsp*47 gene. Within the area of overlap between the fragments there is no shared restriction enzyme sites. PCR based site directed mutagenesis was used to both amplify and ultimately join the two cDNA fragments into one continuous reading frame coding for the human Hsp47 protein. Based on the published sequence data and our choice of cloning sites, two sets of primers were designed for PCR. An artificial BamHI site was introduced flanking the 5' end of the coding sequence. An artificial EcoRI site was introduced at the 3' end of the coding sequence, as well as a MscI site within each side of the area of overlap between the two clones to facilitate the joining of the cDNAs. After PCR amplification of the two plasmids, the smaller fragment was MscI restricted and gel purified. This smaller fragment was used as the 5' primer on the 1300 bp fragment as template and thus generated the first reported expressible gene cassette of human hsp47. The PCR product of this reaction was then directionally ligated into BamHI/EcoRI restricted bacterial protein expression vector pGEX-4T1, and in-vivo amplified from single colonies of transformed BL21(DE3) bacterial host cells.

Human huhsp47 was cloned without its signal peptide "MRSLLLGTLCLLAVALA". pGEX-4T bacterial protein expression system was used for IPTG induction to overexpress Hsp47 and to obtain a fusion protein of human Hsp47 and N-terminally tagged gluthathione-S-transferase (GST). The latter allowed use of a one-step affinity purification protocol on GST substrate columns with crosslinked reduced gluthathione and elution through competitive binding of this fusion partner with free, soluble reduced gluthathione. GST itself has been shown to be non-toxic in most cellular assays and did not increase or reduce target cytotoxicity in the $^{51}$Cr release assays. Purified recombinant huHsp47 protects EC in a dose responsive fashion.

A Kozak initiation sequence and a functional secretion signal for optimal eukaryotic expression was introduced by a combination of PCR and restriction enzyme cloning, using the eukaryotic baculovirus protein expression vector pMel-Bac as template for the Kozak and the mellitin secretion leader sequences. Selection of the strong CMV$^{IE}$ promoter was done to ensure a high level of protein expression. The BFA treatment of EC resulted in a marked increase of secreted, extracellular Hsp47, despite the presence of an ER-retention signal "RDEL" on Hsp47. As detailed above, BFA treatment leads to Ca$^{2+}$ flux pertubation of the ER compartment which results in dysfunctional ER retention of KDEL/RDEL-proteins, including Hsp47. To direct Hsp47 towards secretion in absence of BFA treatment, we deleted the "RVEL" sequence of Hsp47 in one of the constructs. Consistent with this concept, transfection of this construct results in increased secretion of Hsp47 protein and a higher (total) level of protection of EC from CIK, than mediated by the mostly ER retained form which only becomes transported to the cell surface via co-transport with collagen types I and IV (Hughes et al., Eur. J. Biochem. 163:57-65 (1987)). The expression of Hsp47 and procollagen I were found to be tightly linked (Clarke et al., J. Cell Biol. 121:193-199 (1993)).

Further PCR directed cloning of hsp47 into both pro- and eukaryotic protein expression vectors using eGFP-protein fusion tags enabled us to perform FACS analysis with full-length and truncational mutant hsp47 gene cassettes. FACS analysis with full-length eGFP-Hsp47 protein stained a 26% subset of mature CIK.

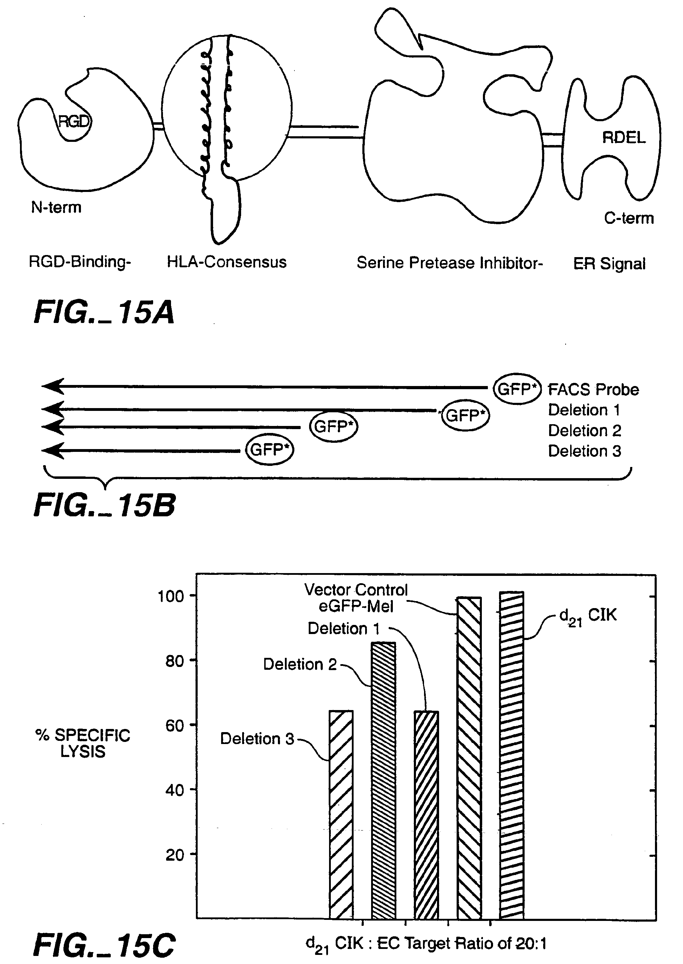

Comparative analysis of the deduced peptide sequence of the human hsp47 gene and the available published data cited above (Clarke, et al., Biochem Biophys Acta 1129:246-248 (1992)), indicate the presence of at least three distinct functional protein domains. We found one additional domain presented in this work. Hsp47 can be thus be depicted in a simplified fashion as a four domain protein (see Figure 15A). Through PCR with staggered primers and subsequent cloning into pro-and eukaryotic protein expression vectors, the protective function of truncated forms of Hsp47 was compared. See Figure 15C. The truncations were chosen to delete hsp47 -gene fragments according to its presumed domain structure. See Figure 15B.

By sequence homology, Hsp47 is a serine protease inhibitor with theoretical specificity for lysine at the active site of incoming serine proteases. Serpins act as "bait" representing cleavable substrates which form a stable instead of a transitory covalent bond with the active center of serine proteases. Those then do not become released any more, thus blocking the active site of the serine protease they reacted with. Thus, serpins inactivate serine proteases in a stoichiometric ratio of 1:1. Despite the sequence relationship, no substrate serine protease has so far been identified for Hsp47. Some authors expect the serpin-domain to be nonfunctional because its "active site" amino acids are modified (Hirayoshi et al., Mol Cell Biol 11:4036-4044 (1991)). We mutagenized Hsp47 in truncational analysis across the serpin domain to evaluate whether a highly specific serine protease of the granule contents of CIK like granzyme A, but were not able to demonstrate a function of Hsp47 as an irreversible inhibitor of attacking granzymes in BLT esterase or SDS-PAGE gelshift assays (data not shown). Our data is paralleled by work with purified murine Hsp47 which failed to inhibit major serine proteases in BLT esterase assays in vitro (Davids et al., Bioorganic Chemistry 23:437-438 (1995)). In $^{51}$Cr-release assays, deletion of the serpin domain led to loss of function of the affected truncation mutants. However, as further deletions of the gene re-established full function to the smaller truncated Hsp47 mutants, expressing then little more than the H1,A-A2-consensus region discussed below, we assume that the loss of function of the C-terminal kuncation of Hsp47's serpin domain causes a conformational change in the protein which normalizes with further truncation.

The second domain is the short C-terminal ER retention signal "RDEL." which we discussed above.

The third domain is the functionally evident collagen/RGD binding domain. So far it has not been directly localized. Truncation of either the C-terminal 32 amino acids or the N-terminal 34 amino acids of murine Hsp47 reportedly reduce the gelatin binding activity of mHsp47 (Davids et al., Bioorganic Chemistry 23:437-438 (1995)). It is however not clear at present, whether these effects are secondary to conformational changes in the Hsp47-truncational mutants or whether they indicate the exact location of the functional RGD-binding domain. Significant sequence homology of mHsp47 exists with the crystallized collagen binding human protein C inhibitor (hPCI). This allowed for one attempt to theoretically model the three dimensional structure of Hsp47 and to predict a RGD binding loop at the C-terminal end of the protein (Davids et al., Bioorganic Chemistry 23:437-438 (1995))

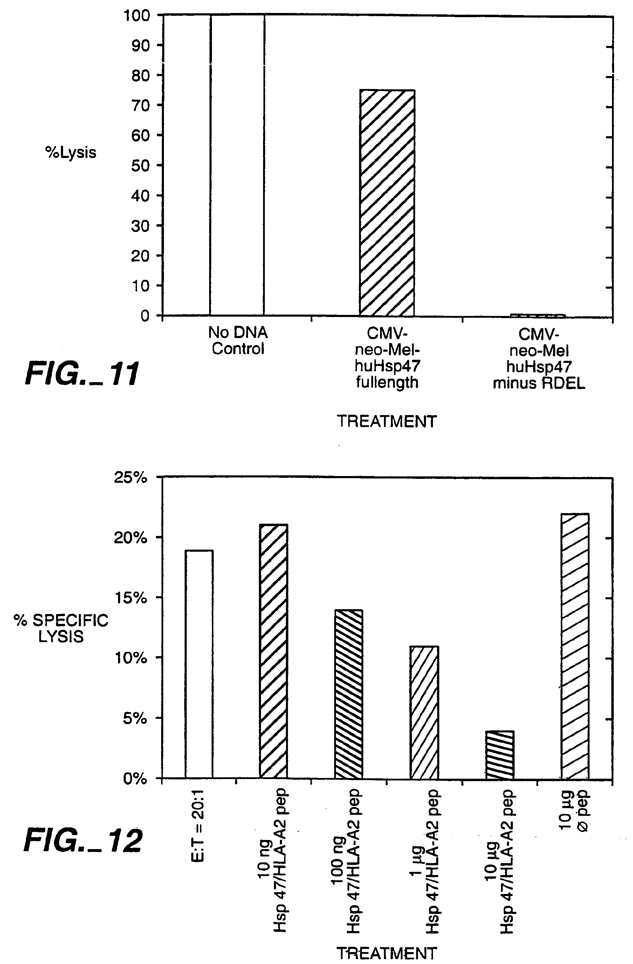

The fourth domain contains an alpha helical stretch closely resembling the peptide binding groove flanking $\alpha_2$-helix of the $a_2$-domain of HLA-A2 and related MHC I proteins. This HLA-A2 consensus peptide was synthesized. This peptide is capable of protecting EC from CIK mediated killing in a dose responsive fashion (Figure 12). An immunoprotective domain of Hsp47 therefore contains a relatively small peptide domain which by itself is capable and sufficient to account for the protection of EC from non-MHC restricted killing.

We postulate that Hsp47 and the deduced peptide mediate protection of EC through a non-kill signal according to the paradigm of MHC I and the "missing self hypothesis" (Karre, Semin Cancer Biol 2:295-309 (1991); Karre et al., Nature 319:675-678 (1986); Ljunggren et al., Immunol Today 11:237-244 (1990)). Such a relationship has been described for killer inhibitory receptor (KIR) pathways (Höglund et al., Immunological Reviews 155:11-28 (1997)). It appears possible that a non-kill signal is elicited in CIK cells through a yet to be identified counterreceptor recognizing qualitative and/or quantitative presence of the collagen chaperone Hsp47 in the vicinity of collagen deposition. Such a model could allow for a mechanistic explanation of tumor surveillance by CIK and other activated NK and T-cell populations *in-vivo:* Hsp47 is co-secreted as collagen chaperone by all cells actively depositing extracellular matrix containing collagens I or IV (Brewer et al., Embo J. 16:7207-7216 (1997)). This secreted Hsp47 can bind to ubiquitous fibronectin consensus sequences (RGD) on fibronectin, osteonectin heparin and collagen molecules in the vicinity of the cell surface of the secreting cell (Nakai et al., Biochem Biophys Res Comm 164:259-264 (1989)). Very recently it was reported that Hsp47 binds to tetraspanin CD9 on the surface of cells (Hebert et al., J. Cell Biochem 73:248-258 (1999)). Tetraspanins are important membrane signal transducing molecules involved in the shaping of the T-cell repertoire (Levy et al., Annu Rev Immunol 16:89-109 (1998)). A related tetraspanin, CD82 was recently reported to bind MHC class I molecules and to interfere with the KIR mediated non-kill signals of NK cells (Lagaudriere-Gesbert et al., J. Immunol 158:2790-2797 (1997)). Lack of adequate MHC I expression level leads to killing by default according to the "missing self hypothesis" in the context of NK target surveillance. In a similar fashion, lack of adequate collagen production by metastatic cells and their vasculature lacking contributory extracellular matrix (EM) production to their tissue of metastasis could thus lead to a lack of non-kill signal mediated by Hsp47 in the context of CIK, NK and T-cell mediated metastatic tumor surveillance.

Similarly, the physiologically aberrant vascular beds of tumors are characterized by fenestrated basal membranes and lack of normal EM production. In healthy individuals, only the ellipsoid meshwork of the spleen and liver have EC with fenestrated basal membranes. Those EC anchoring on physiologically fenestrated basal membranes are reported to have unusually high Hsp47 amounts (Kasai et al., Cell Tissue Res 281:135-141 (1995)). The higher CIK cytotoxicity towards freshly confluent versus 5-day confluent EC *in-vitro* may represent lack of surface localized Hsp47 non-kill signal secondary to still incompletely laid down basement membrane after freshly seeding of TC flasks (Sauk et al., Biochem Biophys Res Comm 172:135-142 (1990)). A Hsp47 mediated non-kill signal theory could provide a mechanism for CIK mediated *in-vivo* screening of tissue resident cells for extracellular matrix production and collagen production. According to this theory, detection and destruction of metastatic solid tumor cells would be the result from the latter lacking collagen and Hsp47 production. Additional support to this concept comes from reports that Hsp47 is down-regulated in cells undergoing oncogenic transformation (Clarke et al., J. Cell Biol 121:193-199 (1993); Hirayoshi et al., Mol Cell Biol 11:4036-4044 (1991)). In *in-vivo* studies, comparing a wide range of solid tumors, it was further found that Hsp47 becomes increasingly less expressed, as those tumors become more malignant and screening for Hsp47 levels was suggested to clinically follow tumor progression in cancer patients (Morino et al., In Vivo 8:285-288 (1994)). Metastatic tumor nodules and their Hsp47 deprived vicinity thus could represent prime targets for cellular *in-vivo* tumor surveillance. EC lining the fenestrated basal membrane found only in tumor neo-angiogenic vasculature represent by itself an additional target leading to specific attack and subsequent necrosis of tumor vascularization. Our SCID/hu-melanoma CIK results are fully consistent with this theory.

Blocking antibodies to KiR or p40 do not enhance the killing of tumor or endothelial targets. The involvement of other killer inhibitory receptors however remains possible.

Previous studies demonstrated by FACS analysis that >95% of bulk generated CIK express CD3, TcR$\alpha$/$\beta$ > TcR$\gamma$/$\delta$ and CD8 > CD4 (Lu et al., J. Immunol 153:1687-1696 (1994)). Blocking antibody studies demonstrate that none of these molecules are functionally involved in the cytotoxicity against either EC or tumor targets (Table 2).

TABLE 2

CIK Cytotoxicity Blocking Studies with Monoclonal Antibodies

| Molecule Class: | Antibody Against: | > 15% Reduction of EC Lysis | > 15% Reduction of Tumor Lysis |
|---|---|---|---|
| Control | Irrelevant Isotype | - | - |
| MHC | MHC I (W6/32) | - | - |

(continued)

CIK Cytotoxicity Blocking Studies with Monoclonal Antibodies

| Molecule Class: | Antibody Against: | > 15% Reduction of EC Lysis | > 15% Reduction of Tumor Lysis |
|---|---|---|---|
| T-Cell Markers: | TCR $\alpha/\beta$ | - | - |
| | TCR $\gamma/\delta$ | - | - |
| | CD 2 (LFA-2) | - | - |
| | CD 4 | - | - |
| | CD 8 | - | - |
| NK-Cell Markers: | CD 16 | - | - |
| | CD 56 | - | - |
| | p40 (via MAb NKTA 255) | - | - |
| | NKB1-Kir (via MAb DX 9) | - | - |
| Cell Adhesion Molecules, $\beta2$ intergrins | ICAM-1 (CD 54) | - | + |
| | LFA-1 (CD 18/11a) | - | + |
| | CD18 (LFA-1 $\beta$-chain) | - | + |
| | CD 11a (LFA-1 $\alpha$-chain) | - | + |
| | CD 11b (Mac-1 $\alpha$-chain) | - | - |
| | CD 11c (CR 4 $\alpha$-chain) | - | - |
| Other CAMs | CD 34 | - | - |
| | PECAM-1 (CD 31) | - | - |
| | VAP-1 | - | - |
| | N-Cadherin | - | - |

Blocking Mab NK-B 1 and NKp40 were also tested in $^{51}$Cr release cytotoxicity assays. Functional involvement of either molecule in CIK mediated cytotoxicity against tumor or EC targets has not been shown. These receptors were assayed because expression of these NK markers is reported to occur on non-classical MHC I restricted cytotoxic T-cell (D'Andrea et al., J. Immunol 155:2306-2310 (1995); Gumperz et al., J. Exp. Med. 181:1133-1144 (1995); Lanier et al., J. Immunol 153:2417-2428 (1994); Lanier et al., Immunol Today 17:86-91 (1996); Phillips et al., Immunity 5:163-172 (1996); Raulet et al., Cell 82:697-700 (1995); Soderstrom et al., J. Immunol 159:1072-1075 (1997)).

CIK can be used *in vivo* without co-injection of IL-2 (Lu et al., J. Immunol 153:1687-1696 (1994)). This is a significant difference to past and current treatment of patients with IL-2 activated CD56+ cells, IL-2 alone (Yang, Cancer 76:687-694 (1995)) or a combination thereof (Phillips, Journal of Clinical Oncology 5:1933-194 (1987)). Whereas the latter approaches reported a significant risk for the development of potentially fatal vascular leak syndrome (VLS), VLS was not observed in the *in-vivo* tumor purging models with SCID/hu mice. Nor was there significant CIK infiltrates in grafted normal human skin.

The reliable expansion and generation of highly specific anti-tumor activity is a sine qua non for any adoptive immune therapy cell production intended for clinical use. High dose $Th_1$-type hormonal stimulation of $\alpha/\beta$ T-cells with IFN-$\gamma$, OKT-3 and IL-2 results in CIK AI cells with superior cytotoxicity against malignant hematopoietic and solid tumor targets. The use of turbine agitated bioreactors proves to be a plus, resulting in rapid, safe and improved selective expansion of the cytolytic CD3+56+ subset. Yields of $10^{11}$ cells are routinely obtained from a single buffy coat source. This is a sufficient number of effector cells for high dose and/or repeat treatment schedules in their clinical application. CIK can be generated from pure T-cell sources, maintained in long-term culture, cryo-preserved, and applied *in-vitro* and *in-vivo* without co-administration of IL-2. These characteristics make CIK superior choices for adoptive immune therapy of cancer as compared to LAK NK derivatives.

**Example 1**

*In vitro* Cell Culture

Generation and Maintenance of CIK cells:

Whole venous blood from healthy community donors, umbilical cord blood and buffy coats obtained by the Stanford Blood Center served as source of peripheral blood lymphocytes (PBL). PBL cells were isolated using Ficoll-Hypaque (Pharmacia Fine Chemicals, Uppsala, Sweden) density gradient centrifugation. PBL were resuspended in RPMI 1640 (GIBCO-BRL/Life Technologies, Grand Island, NY) containing 50 $\mu$m $\beta$-mercaptoethanol (ME), 100 IU penicillin-G ml-1, 100 IU streptomycin ml-1 and 10% FCS (all: Sigma Chemical Co., St.Louis, MO) at a density of 0.5-2x10^6 cells/ml. Enriched populations of large granular lymphocytes (LGL) and T-cells were obtained by subsequent exclusion of plastic and nylon wool adherent cells. Source LGL were cultured in a humidified incubator with 5% carbon dioxide at 37 °C. Hormonal stimulation consisted of addition of recombinant human interferon gamma (rhu g-IFN) (a kind gift of Genentech, South San Francisco, CA) at 1000 IU ml-1 at the start of culture day d0, subsequent stimulation on day one (d1) with soluble anti-human CD3 monoclonal antibody OKT-3 (derived from hybridomas by the American Tissue Culture Collection, Rockville, MD) at 50 ng per ml and recombinant human interleukin-2 (rhuIL-2) (a kind gift of Cetus/Chiron, Emeryville, CA) at 300-500 IU per ml. Cultures were maintained by addition of 50% fresh medium and 300-500 IU rhuIL-2 every 3-4 days for 21 to 28 days. Lytic activity was verified in $^{51}$Cr release cytotoxicity assays against tumor and EC targets at day 14. Preferential expansion of the CIK CD3$^+$, CD56$^+$ double positive phenotype was seen as early as day 10 in culture where an average of 12%-15% of the cells were double positive. Early CIK cultures could be further enriched by via a CD56-magnetic bead column to generate cultures which averaged 60% CD3$^+$ CD56$^+$ double positive phenotype (see Figure 2). For single cell CIK cloning T cells were purified from source LGL by FACS sorting for presence of CD3 or CD56 and absence of CD 16 as described below. Cells were sorted at a mean density of 0.6 cells per well in a 96 well plate and expanded by co-culture with twice irradiated autologous feeder CIK at 0.5x10^6 cells per ml with identical hormonal stimulation as described for bulk culture. Cells were expanded to more than 2x10^6 cells each and then tested for clonal cytotoxicity as described below.

For large scale production of single donor CIK, healthy donor apheresis products were treated as above to isolate LGL. LGL were cultured at 37 °C with identical hormonal stimulation as above, but at a tenfold density of 5-10 x10^6 cells/ml in turbine/impeller agitated bioreacters of 500 ml, 1,000 ml (Nalgene, Rochester, NY) and pO2 and pH controlled 15,000 ml (Chemap, Völketsvil, Switzerland) capacity. Use of 15L bioreactors allowed expansion up to 3x10$^{11}$ cells from a single donor with the expansion of the CD3$^+$ CD56$^+$ cells exceeding 600 fold.

EC Culture, Generation and Maintenance of EC:

Freshly isolated single donor human umbilical chord endothelial cells (HUVEC) were a kind gift from Drs. J. Murphy and J. Alvarnas, (Stanford University Div. Hematology. Stanford, CA). Pooled donor HUVEC were commercially obtained (ATCC, Rockville, MD). HUVEC were cultured on gelatin (Sigma Chemical Co., St. Louis, MO) coated tissue culture flasks, 6 and 24 well plates (Corning, Corning, NY) in media TC-199 or Ham's F12-K, each supplemented with 100 IU penicillin-G per ml, 100 IU streptomycin per ml, 5 $\mu$g porcine heparin per ml, 20% FCS (all: Sigma Chemical Co., St. Louis, MO), and 80 $\mu$g endothelial mitogen per ml (Biomedical Technology Inc., Stoughton, MA) in a humidified incubator with 5% carbon dioxide at 37 °C. Upon reaching 90% confluence HUVEC were passaged using 0.25% trypsin and 0.02% EDTA in Ca2+Mg2+ free phosphate buffered saline (PBS).

Culture of EC on microcarrier beads was performed on 90 $\mu$m and 150 $\mu$m diameter spherical collagen coated acrylic cytocarriers (Kontes, Vineland, NJ) in magnetic stir bar agitated media TC-199 or Hams-F12K, each supplemented with 100 IU penicillin-G per ml, 100 IU streptomycin per ml, 5 $\mu$g porcine heparin per ml and 20% FCS (all: Sigma Chemical Co., St. Louis, MO), and 80$\mu$ g endothelial mitogen per ml (Biomedical Technology Inc., Stoughton, MA) in a humidified incubator with 5% carbon dioxide at 37 °C.

Generation and Maintenance Tumor Target Cell Lines:

The hematopoietic tumor cell lines SU-DHL, OCI-Ly8, K562 and lymphoblastoid cell lines AMK (a kind gift of Dr. A, Krensky, Stanford University, CA) and RDL (a kind gift of Dr. R. Lopez, Stanford University, CA) were cultured in RPMI 1640 medium (GIBCO-BRL/Life Technologies, Grand Island, NY) containing 50 $\mu$m $\beta$-mercapto-ethanol (ME), 100 IU penicillin-G per ml, 100 IU streptomycin per ml and 10% FCS (all: Sigma Chemical Co., St.Louis, MO) at a density of 0.5-2x10^6 cells per ml in a humidified incubator with 5% carbon dioxide at 37 °C.

Melanoma cell lines WM 9 and 1205 LU (a kind gift of Dr. M. Herlyn, Wistar Institute of Anatomy and Biology, Philadelphia, PA) were maintained on tissue culture treated flasks (Beckton Dickinson, San Jose, CA) in MCDB 153 medium (Life Technologies, Grand Island, NY) supplemented with 5 μg per ml insulin and 2% FCS in a humidified incubator with 5% carbon dioxide at 37 °C.

The constituently cytoplasmic luciferase expressing cervical carcinoma cell line HeLaluc (a kind gift of C. Contag, Stanford University, Stanford, CA) was cultured in DMEM medium (GIBCO-BRL/Life Technologies, Grand Island, NY) containing 50 μm β-mercapto-ethanol (ME), 100 IU penicillin-G per ml, 100 IU streptomycin per ml and 10% FCS (all: Sigma Chemical Co., St.Louis, MO) in a humidified incubator with 5% carbon dioxide at 37 °C.

CTL cell-line AJY and specific CTL target cell-line AJ (lymphoblastoid) were kindly provided by the laboratory of A. Krensky (Stanford University, Stanford, CA).

## Example 2

### Cloning and Expression of the Hsp47 Gene

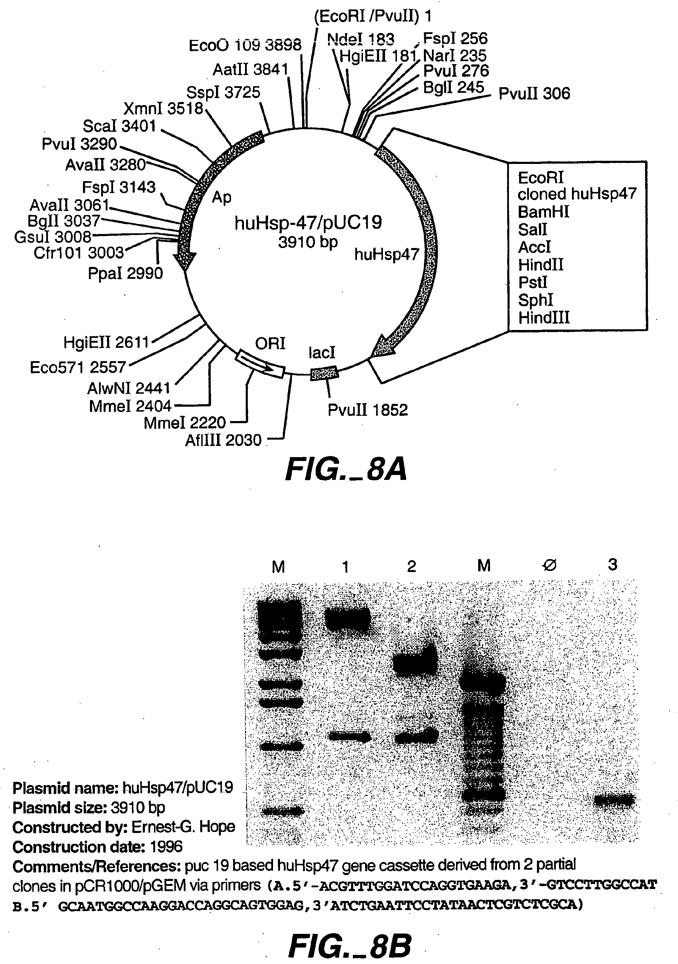

Partial huHsp47 gene fragments derived by RT-PCR (a kind gift of Drs. Sanwal, Ontario, Canada) were amplified *in vitro* and artificial cloning sites introduced by PCR. These fragments correspond to nucleotides in Figure 1A. The nucleic acid encoding the amino terminal 39 amino acids of Hsp47 (excluding the signal sequence and first amino acid of the mature protein) was amplified with the following primers: 5' primer ACGTTTGGATCCAGGTGAAGA, 3' primer GTCCTTGGCCAT. The 5' primer incorporated a Bam HI site to facilitate cloning into further vectors. The 3' primer incorporated an Mlu NI site to facilitate fusing the nucleic acids encoding the amino and the carboxy terminal portion of the protein. The nucleic acid encoding the carboxy terminal 360 amino acids was amplified with the following primers: 5' primer GCAATGGCCAAGGACCAGGCAGTGGAG, 3' primer ACGCTCTGCTCAATATCCTTAAGTCTA. The 5' primer incorporated an Mlu NI site to facilitate fusing the two portions of the gene into one continuous reading frame and the 3' primer incorporated an Eco RI site to facilitate cloning into further vectors. Standard PCR conditions generally known to those of skill in the art were used to amplify the two fragments from the starting clones (see Figure 8). The resulting amplification products were digested with Mlu NI, purified and ligated to each other. The resulting nucleic acid was further digested with Bam HI and Eco RI and the huHsp47 gene cassette was directionally cloned using standard recombinant DNA techniques into pUC 19 (Pharmacia, Uppsala, Sweden) which was used for construction purposes. The resulting plasmid, pUC/huHsp47, was amplified in recA bacterial host strain DH5a (Life Technologies, NY). The sequence of the cloned gene cassette was verified via fluorescent sequencing on an ABI sequencer (ABI,) using T7 DNA polymerase (Pharmacia, Uppsala, Sweden).

The huHsp47 gene cassette was PCR amplified using an alternative set of primers which results in a BamHI-EcoRI expressible gene cassette for bacterial protein expression. The expressible gene cassette was cloned into the pGEX 4T$_1$ vector (Pharmacia, Uppsala, Sweden), which produces a GST-fusion protein under the control of the IPTG inducible laq$^q$ promoter, and grown in the bacterial host strain BL21(DE3) (Stratagene, La Jolla, CA).

Expression of recombinant GST-fusion protein in bacterial host cells was induced with 20 mM IPTG via the lactose promoter laq$^q$ and overexpressed proteins purified via affinity chromatography on glutathione sepharose 4B (Pharmacia Biotech, Uppsala, Sweden), eluted at room temperature with 5-20 mM reduced glutathione in 50 mM Tris-HCl buffer at pH 8.0, analyzed quantitatively by Bradford assay and qualitatively by SDS-PAGE. The elution buffer was exchanged with PBS at pH 7.4 via PD-10 (Pharmacia Biotech, Uppsala, Sweden) gel filtration prior to use. When removal of the GST-fusion partner was desired, cleavage with 10 IU of thrombin (Sigma, St. Louis, MO) per μg fused GST moiety was performed for 10-12 hours at 37 °C using a thrombin-cleavage site present in the linker amino acids between GST and the recombinant protein of interest. Routinely 5-10 mg of recombinant huHsp47 fusion protein were obtained per 4 liters of BL21(DE3) host cells transformed by pGEX-4T-Hsp47 via glutathione affinity FPLC. Purified huHsp47 was used in [51]CR release cytotoxicity assays described above.

To dissect the role each domain of huHsp47 plays in protecting EC from CIK induced cytotoxicity, domain specific deletion mutants of huHsp47 were generated via PCR with nested primers based on the sequence disclosed in Figure 1 and previously published huHsp47 sequences. HuHsp 47 has at least four distinct functional domains. Beginning at the amino-terminus of the protein they are: a collagen/RGD binding domain, a domain with homology to the α-2 domain of human HLA-A2 molecules, a serine protease inhibitor (serpin) domain and an ER retention signal RDEL domain. Three specific deletions were generated. Deletion 1 removed the RDEL domain, deletion 2 removed the RDEL and serpin domains and deletion 3 removed the carboxy-terminal 150 amino acids including the serpin and RDEL domains. The same 5' primer was used in PCR reactions to generate all three deletion mutants. The 5' primer sequence is: CGGAATTCTGGCCGAGGTGAAGAAACC. The 3' primer used to generate the RDEL deletion mutant was the same primer used to generate the huHsp47-GFP fusion protein and has the sequence: AGTTCCCACTGTTCTACGACCTAGGGC. The deletion 2 3' primer is: AACTCAACCTGTGTCTAGACCTATGGGC. The deletion 3 3' primer is: ACGCGCTGCTCCTCCACGACCTAGGGC (see Figure 14). The 5' and 3' primers incorporated BamHI and EcoRI restriction enzyme sites respectively to facilitate subsequent cloning steps. The 5' and individual 3' primers were mixed with the pUC/ huHsp47 plasmid containing the huHsp47 gene cassette and nucleic acids encoding each deletion mutant were PCR amplified in separate reactions. The PCR products were purified, digested with BamHI and EcoRI and ligated into either the pGEX-4T$_1$ vector to create huHsp47deletion-GST fusion proteins for cytotoxicity assays (see below) or pEGFP-NI, which also contained the mel secretion signal described above, to create eGFP-Hsp47deletion fusion proteins for use as FACS probes.

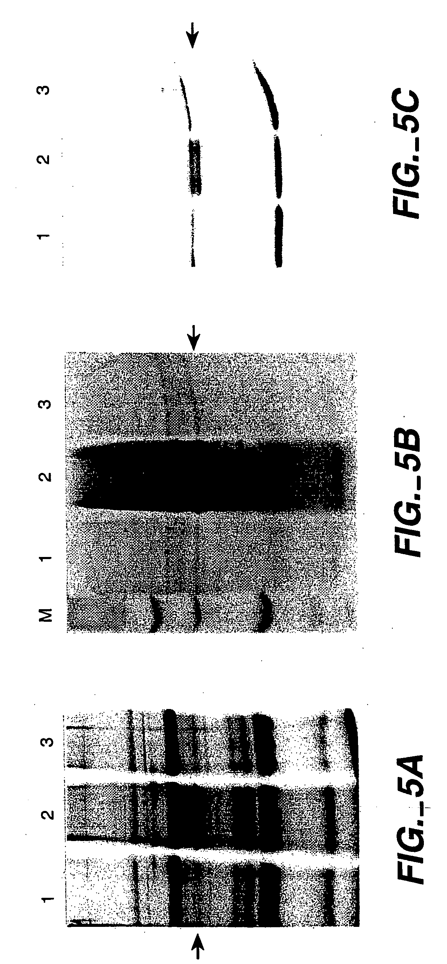

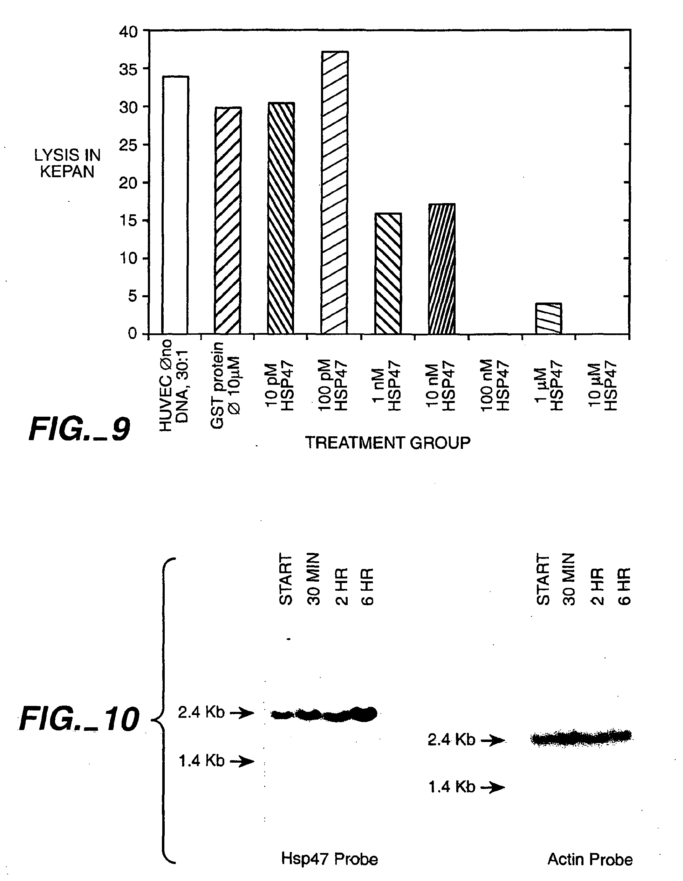

Northern analysis of huHsp47 RNA expression was carried out via $\alpha^{32}$P-ATP nick translation labeling of the agarose gel purified full-length huHSP47 cDNA as probe and human beta action (a kind gift of Dr. S.N. Cohen) cDNA as internal control probe. Total RNA was harvested at 0 hour, 2 hour and 6 hour time points from EC undergoing BFA treatment. RNA for each time point was purified, denatured, subjected to formamide flatbed agarose gel electrophoresis, transferred to nitrocellulose, prehybridized, hybridized with the $^{32}$P-labelled probes, washed under stringent conditions and autoradiographed without intensifier screens at -80 °C using techniques well known to those skilled in the art (see Sambrook et al. Molecular Cloning, A Laboratory Manual, second edition, Cold Spring Harbor Laboratory Press, 1989). Results indicate that BFA treatment does not affect the expression of the housekeeping gene beta action, but that BFA treatment does upregulate huHsp47 gene expression over the period evaluated. See Figure 10. These data are consistant with the observed increase in Hsp47 protein seen in BFA treated cells and isolated by immunoprecipitation experiments. See Figure 5.

Protease inhibitor free production of rhuHsp47 for granzyme A assays was carried out by growing protease free transformed BL21 (DE3) bacteria in amp selective NZCYM medium using casein acidic hydrolysate in lieu of tryptic casein amino acids as nutritional peptide source for the host bacteria undergoing IPTG induction. To minimize incubation times, protein extracts were batch incubated on glutathione resin and initial removal of non GST proteins was carried out with a modified spin column approach. The remainder of the protocol was unchanged, thrombin was not utilized.

**Example 3**

Cytotoxicity Assays

$^{51}$Cr-Release Assay

Target cells were metabolically labelled with $^{51}$Cr (Dupont- New England Nuclear, Boston, MA) by incubating 1x10$^6$ cells in 300 mCi $^{51}$Cr at 37 ° C for 1-1.5 hours. The labelled cells were washed three times with phosphate buffered saline (PBS) containing 0.1% bovine serum albumin. The labelled cells were distributed in flat-bottomed 96 well microtiter plates at a concentration of 2x10$^4$ cells/well in triplicate. Effector (CIK) cells were added at the indicated ratios. Mabs were added prior to the addition of effector cells, and incubated for 15-30 minutes at room temperature. The final volume of the assay mixture in each well was 0.2 ml. After 4 hours at 37° C, the cells were collected by centrifugation and an aliquot of the supernatant was counted in a gamma counter (Micromedic Systems, Horsham, PA). The percentage of specific $^{51}$Cr release was calculated according to the following equation:

$$\% \ specific \ ^{51}Cr \ release = \frac{(test \ release) - (spontaneous \ release)}{(maximal \ release) - (spontaneous \ release)} \ x \ 100\%$$

Spontaneous release was obtained by incubating the labelled cells in complete medium alone and maximal release by treatment of the cells with 1% NP-40.

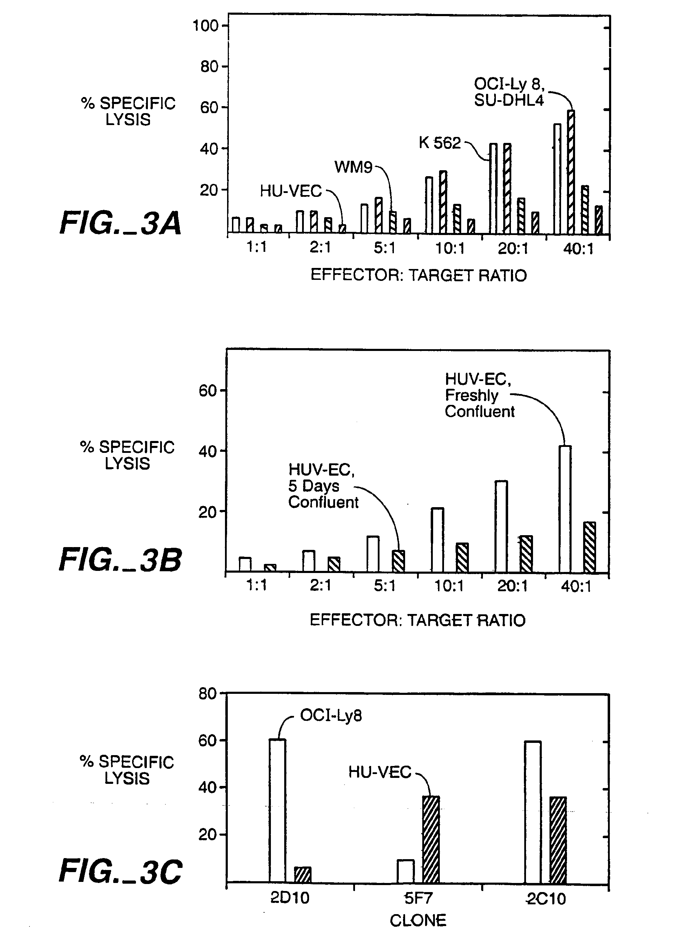

The anti-tumor activity of adult and umbilical cord derived CIK cells were compared in cytotoxicity assays. The cytotoxicity results of the CIK cells derived from both sources were superimposable against B-cell lymphomas OCI-Ly8 and SU-DHL4/LAM53 and myeloid leukemia target K562. Both sets of CIK cells displayed marginal cytotoxicity against melanoma WM9 *in vitro.* Both sets of CIK cells were cytotoxic against freshly confluent HUVEC and less so against EC monolayers confluent for longer periods of time (see Figure 3).

Figures 3A, 3B and 3C describe 4 hour $^{51}$Cr release cytotoxicity assays with d$_{21}$ CIK and various targets. CIK were derived from PBMC by culture in complete RPMI supplemented by the following: At d$_0$: IFN$_{-g}$ at 1000 u ml$^{-1}$. At d$_1$: 5 ng soluble OKT-3 and 500 u IL-2 ml$^{-1}$. At d$_5$, d$_9$, d$_{13}$, d$_{17}$ and d$_{21}$: 500 u IL-2 ml$^{-1}$. All cells were in a humidified incubator with 5% CO$^2$ at 37°C. The hematopoietic tumor lines K562, OCI-Ly8, and SU-DHL4 were maintained in complete RPMI medium; melanoma line W9 was cultured in MCDB 153 medium supplemented with 5 $\mu$g ml$^{-1}$ insulin and 2% FCS. HUV-EC were obtained from the ATCC. They were grown on 2% gelatin coated 24 well plates in TC-199 or Ham's F12-K medium, each supplemented with 20% FCS, 5 $\mu$g porcine heparin ml$^{-1}$ and 80 $\mu$g endothelial mitogen ml$^{-1}$. Routine tryptic passaging was carried out when HUVEC reached more than 80% confluency; $^{51}$Cr release assays were routinely performed at the time point of fresh confluency.

Figure 3A is a cytotoxicity profile of adult and cord blood CIK d$_{21}$ CIK shows high cytotoxicity against B-cell lymphomas OCI-Ly8 and SU-DHL4 (blue), as well as erythromyeloid leukemia K562 (yellow). Moderate cytotoxicity exists against nodular melanoma line WM9 (red) and freshly confluent HUV-EC (dark green).

Figure 3B shows the effect of EC culture duration after reaching confluency on the EC sensitivity to $d_{21}$ CIK lysis: freshly confluent EC (dark green) are lysed significantly more readily than are EC cultured for 5 additional days after reaching confluency (light green).

In Figure 3C clones of CIK were generated via plating of a mean of "0.6 cells per well" of a 96 well plate; then expanded by co-culture with twice irradiated, autologous feeder cells at $0.5 \times 10^6$ cells ml$^{-1}$, and with identical hormonal stimulation as described above for bulk cultures. After individual clones expanded to more than $2 \times 10^6$ cells each, they were tested for clonal cytotoxicity profile at a E:T ratio of 5:1. Target specificity of CIK is determined at the clonal level.

Expanded CIK single cell clones were tested to determine if subpopulations of cells exhibited target specificity in cytotoxicity assays performed as described above. Target specificity was observed, was line specific and was directed against tumor cells only, EC cells only or against both targets, the latter being the most common. The 2D10 CIK cell line tested displayed cytotoxicity against the OCI-Ly8 tumor cell line but not the HUVEC cell line. The 5F7 CIK cell line displayed cytoxicity against HUVEC cells but not OCI-Ly8 cells. The 2C10 CIK cell line was cytotoxic against both the OCI-Ly8 and HUVEC cells.

BFA Treatment of Targets:

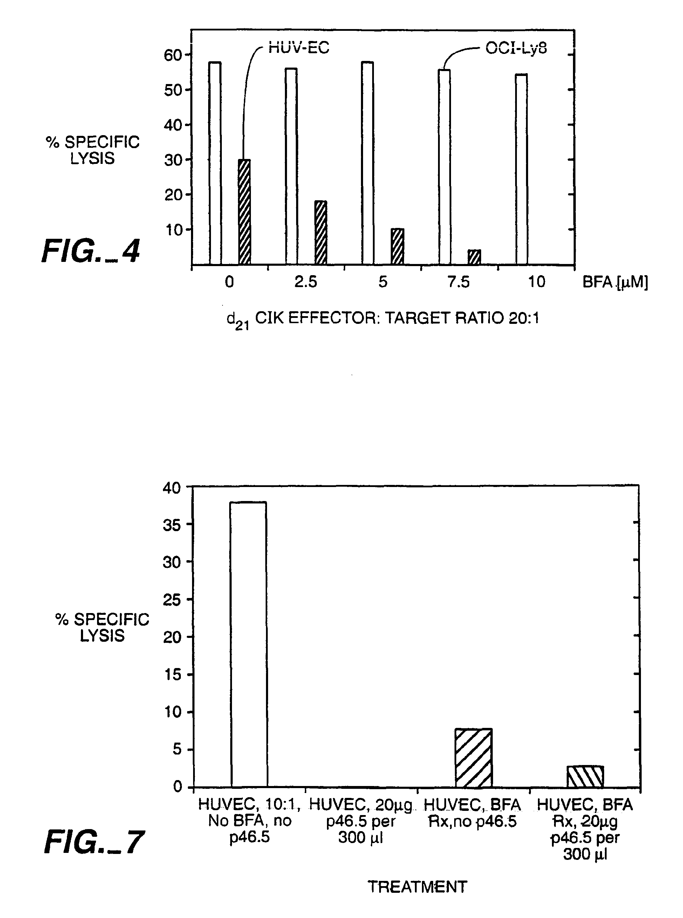

For brefeldin A (BFA) treatment, freshly confluent HUVEC monolayers, nonadherent target cells or CIK cells at $0.5-2 \times 10^6$ cells per ml were incubated with medium supplemented with 0.5-10 $\mu$g of brefeldin A (Böhringer Mannheim GmbH, Mannheim, Germany) per ml medium and incubated for 2-6 hours in a humidified incubator with 5% carbon dioxide at 37 °C. BFA was dissolved at 100 $\mu$g per ml in 70% ethanol and used fresh or stored up to 4 weeks at -20 °C. Prior to cytotoxicity assay the cells were rinsed three times with PBS, and fresh medium not containing BFA was added for the remainder of the assay (Figure 4).

BFA treatment interferes with the intracellular vesicle transport from the endoplasmic reticulum to the proximal and intermediate golgi compartments preventing glycosylation of proteins directed for secretion and active vesicle transport of mature proteins to the cell surface (Lippincott-Schwartz et al., 1990; Lippincott-Schwartz et al., 1991; Lippincott-Schwartz et al.,1989; Orci et al., 1991; Vogel et al., 1993). Brefeldin treatment of endothelial cells leads to up regulation and secretion of a 46.5 KDa protein inhibitor presently identified as Hsp47 by immunodetection with MAB SPA470. Figure 5 shows immunoprotection with Mab N6 of $^{35}$S and biotin labeled HUVEC extracts after 6 hours of BFA induction. Lanes: M = marker, I = wt, 2 = 10 $\mu$M, 3 = $\mu$M BFA.

Figure 5A is a silver stained SDS-PAGE, 10% polyacrylamide gel. Specific detection of p46.5 occurs only at a protective BFA dose. Note heavy and light chains of N6 Mab IgG.

Figure 5B is an auto-radiograph of a 12% polyacrylamide gel SDS-PAGE transferred to an NC membrane. A 46.5 KDa $^{35}$S radio-labeled band is present only at 10 $\mu$M BFA.

Figure 5C demonstrates the positive identification of p46.5: It is huHsp47, immuno-detection with Hsp47 specific Mab SPA 470. Hsp47 migrates in this 12% PAGE with identical $M_r$ of 46,500 as the radio-labeled peptide. The protein bands of p46.5 and huHsp47 are superimposable. Note, that due to the use of mouse IgG Mab N6 for the IP and detection of the primary mouse anti-Hsp47 Mab SPA470 with secondary goat-anti mouse peroxidase Ab, the heavy and light chains of the N6 Mab are also detected in this ECL assay.

BFA treatment of targets was able to suppress the CIK mediated lysis of EC in a dose dependent manner but did not affect the ability of CIK cells to lyse tumor targets (Figure 4). Both HUVEC and OCI-Ly8 cells were incubated with BFA at concentrations of 0, 2.5, 5, 7.5 and 10 $\mu$M for 6 hours as described above, washed, labeled with $^{51}$Cr and used in a cytotoxicity assay with $d_{21}$ CIK cells at an E:T ratio of 20:1. Preincubation of EC targets with 2.5 $\mu$M BFA reduced CIK mediated cytotoxicity by 40%. Larger doses reduced cytotoxicity further and preincubation in 10 $\mu$M BFA was sufficient to completely block CIK mediated lysis of HUVECs.

$^{51}$Cr Release Cytotoxicity Inhibition Assays with Blocking Mabs:

Prior to addition of effector cells 5 $\mu$g of purified, NaN3 free monoclonal antibody (Mab) was added per ml of medium to the target cells and incubated at room temperature (RT) for 15 minutes. Effector cells were added directly to the treated targets. The remainder of the assay was performed as detailed above, at 37 °C in a humidified atmosphere of 5% CO$_2$.

Mabs to MHC class I (W6/32), T-cell markers CD2 (LFA-2), CD4, CD8, TcR$\alpha/\beta$ OR $\gamma/\delta$, and NK markers CD 16, CD56, NK-B 1 (NKTA255) and Nkp40 (DX 9) do not block the cytotoxicity of CIK cells against either tumor or EC targets. Mabs to ICAM-1 (CD54), and LFA-1 (CD 18/11$_a$, LFA-1 $\beta$-chain, LFA-1 $\alpha$-chain) do block cytotoxicity of CIK cells against tumor cells but not EC targets. No other anti-CAM Mab tested (CR 4 $\alpha$-chain, CD 34, CD 31, VAP-1 or N-Cadherin) blocked the cytotoxicity of CIK cells against tumor targets. None of the antibodies tested blocked the cytotoxicity of CIK cells against EC targets indicating that CIK cells interact differently with tumor targets (see Table 2).

<u>$^{51}$Cr Release Cytotoxicity Assays with Protein and Peptide Addition:</u>

Subsequent to $^{51}$Cr labelling but prior to addition of effector cells 10-200 $\mu$g FPLC purified Hsp47 (Example 7) protein or 10 nM to 10 $\mu$M HPLC purified peptide AVLSAEQLR (Example 8), both in sterile PBS at pH 7.4 and diluted to the final concentration with RPMI, were added to targets per ml of medium and incubated for 30 minutes at 37 °C in a humidified atmosphere of 5% $CO_2$. In assays using purified protein, targets were subjected to three subsequent washes with 10 volumes of $Ca^{2+}$, $Mg^{2+}$ and 5% FCS containing PBS prior to providing fresh standard culture medium to the target cells. In assays using purified peptide, the added peptide was present for the entire assay. The remainder of the assay was performed as detailed above, at 37 °C in a humidified atmosphere of 5% $CO_2$.

Incubation of EC with 20$\mu$gm of gelatin-affinity purified (described below) huHsp47 per 300 $\mu$l was sufficient to completely protect the target from CIK mediated lysis (see Figure 7) in an identical fashion to the protection observed with 6h, 10 $\mu$M BFA treatment described above (see Figure 7).

The addition of as little as 1$\eta$M of huHsp47-GST protein was able to reduce EC lysis by 50 %. Pre-incubation with 10 $\mu$M of huHsp47-GST protein completely protects EC from CIK mediated lysis (see Figure 9). The peptide AVLSAEQLR was also able to protect EC from CIK mediated lysis in a dose dependent manner in assays where the E: T ration was 20:1 (see Figure 12). Peptide at a concentration of 1 $\mu$M was able to reduce EC lysis by 50 % and peptide at a concentration of 10 $\mu$M was able to suppress CIK mediated lysis almost completely. The peptide was capable of reducing specific target lysis by less than 10% when used at 10 $\mu$M in a CIK-OCI-Ly8 tumor cell $^{51}$Cr cytotoxicity assay with an E:T ratio of 20:1.

The protective effects of each of the huHsp47 deletion mutants (Fig. 15B) were also analysed. See Fig. 15C. Deletion 1 mutant protein, lacking the RDEL domain, reduced CIK mediated lysis by 40% indicating that the RDEL domain is not involved in protecting the EC from cytotoxicity. Deletion 2 mutant protein, lacking both the serpin and RDEL domains, only reduced CIK mediated lysis by 20% suggesting either the serpin domain is involved in protecting EC from cytotoxicity or the resulting protein is folded aberrantly resulting in a less active form of the protein. Deletion 3 mutant protein, lacking the carboxy-terminal 150 amino acids, was as effective as deletion 1 mutant protein in protecting EC from CIK mediated lysis suggesting that the reduced protection seen with the deletion 2 mutant protein is due to an altered conformation of the protein and is not due to the serpin domain participating in the protection. The deletion 3 mutant protein retains both the collagen/RGD binding domain and the $\alpha$2-HLA-A2 homology domain which contains the AVLSAEQLR peptide sequence.

<u>Protection of Endothelial Cells by Transfection With an Hsp47 Protein Expression Vector</u>

Nucleic acids encoding huHsp47 and fragments were cloned into eucaryotic expression vectors. A nucleic acid encoding a fragment of huHsp47, in which the carboxy-terminal RDEL amino acid sequence is deleted, was PCR amplified from the pUC/huHsp47 plasmid using the following primers: 5' primer CGGAATTCTGGCCGAGGTGAAGAAACC, 3' primer AGTTCCCACTGTTCTACGACCTAGGGC. The amplified product was ligated to the melittin secretion signal and Kozak sequences derived from pMel-Bac (Invitrogen, San Diego, CA) and the resulting fragment was cloned into the multiple cloning site of pEGFP-N1 (Clontech, Palo Alto, CA) using general techniques well known to those of skill in the art. The resulting plasmid, eGFP-Hsp47, was transfected into EC.

Nucleic acids encoding full length huHsp47 and huHsp47$\delta$RDEL were cloned into the pBK-CMV (Stratagene, La Jolla, CA) and expressed in EC. The pBK vector was digested with Nhe I and EcoRI to remove the Lac promotor, most of the MCS and a portion of the Lac Z gene. The mellitin secretion signal peptide and Kozak sequence were PCR amplified from the pMel-Bac vector as described above and ligated into the pBK vector adjacent to the CMV IE promoter. Gene casettes encoding either full length huHsp47 or huHsp47$\delta$RDEL were ligated into the pBK vector adjacent to the the mel signal sequence and the resulting vectors were used to transform EC. The CMV-neo-mel-huHsp47fullength and CMV-neo-mel-huHsp47$\delta$RDEL vectors were purified from host cell cultures containing each vector and used to transfect EC using a low dose lipofectamine (GIBCO/BRL,NY) transfection protocol. The transfected EC were then analysed in CIK cytotoxicity assays.

EC were also co-transfected with a combination of vectors; eGFP-Hsp47, CMV-neo-mel-huHsp47fullength and pCMV-CD20 (a vector expressing CD-20 under the control of the CMV promoter, a kind gift of Dr. S.vHeuvel, Massachusetts General Hospital, Boston, MA) using the low dose lipofectamine transfection protocol. Cells were subjected to FACS analysis 24 hours after co-transfection.

EC targets were used in cytotoxicity assays 24 hours after lipofectamine mediated transfection of either CMV-neo-mel-huHsp47fullength or CMV-neo-mel-huHsp47$\delta$RDEL into previously susceptable EC. Mature $d_{21}$ CIK cells were used at an E:T ratio of 20:1. EC over-expressing full length huHsp47 showed a 25% reduction in the CIK mediated lysis. EC over-expressing huHsp47$\delta$RDEL were entirely resistant to CIK mediated lysis (see Figure 11). Deletion of the RDEL peptide, which serves as an endoplasmic reticulum (ER) retention signal, from huHsp47, allows the protein to be freely secreted and protects the EC targets expressing the protein from CIK mediated lysis.

**Example 4**

Fluorescence Activated Cell Sorting and Imaging

For Fluorescence Activated Cell Sorting (FACS) $10^6$ effector or target cells were washed twice with $Ca^{2+}$, $Mg^{2+}$ and 5% FCS containing PBS buffer, suspended in 50 $\mu$l of this buffer and placed at 0-4 °C in the dark. 1 $\mu$g of FITC-, PE-, Per-CP or Tricolor labelled monoclonal antibody (Beckton Dickinson, San Jose, CA) per staining reaction of $10^6$ cells was mixed with the cells and incubated for 15 to 30 minutes at 0-4 °C in the dark. Stained cells were subjected to three subsequent washes with 10 volumes of $Ca^{2+}$, $Mg^{2+}$ and 5% FCS containing PBS and taken up in 500 $\mu$l of this buffer for analysis or sort by FACScan/FACStar (Beckton Dickinson, San Jose, CA) with excitation at 488 nm and the appropriate channel filters for detection of emission.

Cell staining for immunofluorescence microscopy was carried out in a similar manner and stained cells analyzed with a Axiophot fluorescence camera system (Zeiss, Hanau, Germany).

The huHsp47δRDEL-enhanced green fluorescent protein (eGFP) fusion protein, expressed under the control of the CMV IE promoter, was isolated as described above and used as a FACS probe to search for huHsp47 cell surface binding proteins. FACS analysis of mature $d_{21}$CIK with eGFP-tagged rhuHsp47 as molecular probe was carried out by first staining with PE-Mab and specifically PE-CD56 if desired, then by suspending the washed CIK in 10 $\mu$g eGFP-huHsp47 per ml of PBS, incubating for 15 minutes at 0-4 °C in the dark, removal of unbound protein by centrifugation in a refrigerated eppendorf centrifuge at 1000 rpm at 0-4 °C. Stained cell pellets were either resuspended in 500-1000 $\mu$l of PBS immediately prior to the FACS analysis or suspended in PBS fixans containing 3% (w/v) formaldehyde prior to analysis. After staining, cells were sorted using the FITC channel. 26% of the cells analysed were positive for both eGFP-huHsp47 and PE-CD56 probes indicating that a distinct subset of the CIK cells had receptors for huHsp47.

EC cells co-transfected with eGFP-Hsp47, CMV-neo-mel-huHsp47fullength and pCMV-CD20 using the low dose lipofectamine transfection protocol were analysed 24 hours after co-transfection. Cells were stained with PE-CD20 Mab and analysed for the presence of both the CD-20 antigen and eGFP-Hsp47 using the FITC channel. Over 79% of the cells were positive for both probes indicating that there was an extremely high transfection efficiency.

**Example 5**

Native Protein Purification and Analysis

Protein Extraction from Target Cells

Prior to cell harvest, targets were cultured or treated BFA as described above, and dead cells removed from EC monolayers by washes with $Ca^{2+}$, $Mg^{2+}$ containing PBS, from nonadherent targets via density gradient centrifugation. Cell lysis was performed with ultrasonic cell disruption (Branson, New Brunswick, NJ) in PBS buffer containing 1% Triton-X 100 (Tx-100, Sigma, St. Louis, MO) and complete protease inhibitor cocktail (Böhringer Mannheim, Mannheim, Germany).

For metabolic $^{35}$S methionine/cysteine labelling cells were cultured for 22 hours in cysteine and methionine free RPMI 1640 or Hams-F12K (Irvine Scientific, Santa Ana, CA) media with dialyzed FCS, otherwise as described above, prior to the addition of 100 $\mu$Ci ml-1 of Translabel (PIERCE/Amersham, Amersham, UK) $^{35}$S methionine/cysteine at the start of BFA, control treatment and incubated for the indicated times. Pulse-chase labelled proteins were analyzed by SDS-PAGE, western analysis or autoradiography and silver staining.

Labelling of membrane proteins by biotinylation was carried out after applicable treatments with BFA and subsequent stringent removal of dead cell. Water soluble, lipid insoluble 23-atom linker arm modified biotin analogues NHS-SS-biotin and NHS-LC-biotin (PIERCE, Rockford, IL) were added to a final of 1 mg ml-1 ofPBS and cell monolayers incubated for 30 minutes at room temperature. The reaction was stopped by removal of the biotin labelling reaction and three washes with 10 volumes of 100 mM Tris-HCl, 150 mM NaCl, 5 mM EDTA buffer at pH 7.4 providing excess amine to react with residual NHS-groups.

Purification of NHS-SS-biotin biotinylated proteins was carried out by affinity chromatography on a resin of streptavidin immobilized to 6% crosslinked agarose (Sigma, St. Louis, MO) under UV260nm control (Pharmacia, Uppsala, Sweden). Bound proteins were released from the streptavidin matrix by cleaving the disulfide bond of the 23 atom-SS-biotin linker arm with 100 mM β-mercapto ethanol: This results in elution of the previously biotin-tagged protein leaving the biotin moiety bound to the steptavidin resin. Specifically eluted proteins were analyzed by SDS-PAGE, autoradiography and silver staining. Detection of NHS-LC-biotinylated proteins was carried out by SDS-PAGE, subsequent transfer to solid support membranes and reaction with streptavidin-alkaline phosphatase (AP). Bound streptavidin-alkaline phosphatase was detected via colourimetric BCIP/NBT blot assay. Subsequent autoradiography was carried out with metabolically $^{35}$S methionine/cysteine labelled extracts.

**Example 6**

Generation and Preparation of Antibodies

Generation and preparation of polyclonal antibodies:

Generation of polyclonal rabbit antisera was performed after harvest of non-immune/preserum from three female New Zealand rabbits. One mg of total protein was used per immunization day and animal. Ultracentrifugation derived wild-type (wt) and TM treated lymphoblastoid target cell membrane preparations were used as antigens. Antigens were prepared as emulsion in complete Freud's adjuvant (CFA, Sigma, St. Louis, MO) and injected subcutaneously. This was followed by three booster immunizations at six week intervals as above, but substituting FCA for Freud's incomplete adjuvant (FIA). Titer development was monitored by antigen spot assay of rabbit ear vein blood samples obtained at time points of boosting. Serum was separated from the cell product of collected specimen by coagulation and subsequent centrifugation, aspirated and stored frozen at -20 °C until used.

Preparation of Monoclonal Antibodies:

Limited production of monoclonal N6 antibody in-vivo: Pan-anti-heat shock protein Mab N6 hybridoma (a kind gift of Dr. K Nagata, Kyoto, Japan) was injected intraperitoneally into six-week old female Balb/c mice. Ascitis fluid was harvested, shockfrozen in liquid N2 and stored at -20 °C until needed.

Antibody Purification:

FPLC purification of polyclonal and monoclonal antibodies from serum or ascitis was performed after 7x dilution in PBS and chromatography on Protein A sepharose (Pharmacia, Uppsala, Sweden) with UV260nm control. Bound Ig fractions were eluted with 25 mM glycine buffer at pH 2.5 and rapidly titrated back to pH 7.4 by addition of TRIZMA (Sigma, St. Louis, MO) base. Protein amounts were determined by Bradford assay at 595 nm in a DU-650 spectrophotometer (Beckman, Palo Alto, CA).

Immunoprecipitation was carried out by first preparing preformed immune complexes as follows: Purified specific mouse monoclonal or rabbit polyclonal antibody were incubated in stoichiometric ratio with purified secondary goat anti-mouse or anti-rabbit antibody at a concentration of 10 $\mu$g ml-1 of 150 mM NaCl, 10 mM Tris-HCl, 1% (w/v) NP40, pH 7.8 with gentle agitation for 3 hours at 4 °C. In a separate reaction their respective isotype matched control antibodies were coupled to protein A sepharose beads (Pharmacia, Uppsala, Sweden). The formed immune complexes were harvested by centrifugation at 13,000 rpm in a refrigerated Eppendorf centrifuge (Eppendorf, Hamburg, Germany) for 15 minutes at 4 °C, washed twice with one volume of 500 mM NaCl, 50 mM Tris-HCl, 2% BSA, 0.25% (w/v) SDS, 0.5% (w/v) Tx-100, pH 7.5 and taken up in 120 $\mu$l of 150 mM NaCl, 10 mM Tris-HCl, 1% (w/v) NP40 at pH 7.8. For protein extraction, $2x10^7$ cells per ml of buffer were lysed by sonication in 150 mM NaCl, 10 mM Tris-HCl, 1% (w/v) NP40, 1 mM PMSF, 20 $\mu$g ml-1 trypsin-inhibitor, 5 mM EDTA at pH 7.8. Cell debris was removed by centrifugation at 13,000 rpm for 30 minutes at 4 °C. The lysate was cleared with the isotype matched control antibodies coupled to protein A sepharose beads by agitation for 3 hours at 4 °C and subsequent removal of the beads by centrifugation at 13,000 rpm for 15 minutes at 4 °C. Immunoprecipitation with specific antibody was carried out by adding 30 $\mu$l of cleared protein extract to 200 $\mu$l of 150 mM NaCl, 10 mM Tris-HCl, 1% (w/v) NP40, pH 7.8 and incubating with 10 $\mu$l of preformed specific immune complexes per reaction with gentle agitation for 3 hours at 4 °C. The specific immune precipitate was recovered by centrifugation at 13,000 rpm for 30 minutes at 4 °C, washed twice with 10 mM Tris-HCl at pH 6.8 and analyzed by SDS-PAGE and subsequent silver stain, transfer to solid support and streptavidin-AP detection and autoradiography.

AMK targets were first treated for 16 hours with TM at 5 $\mu$g per ml or left untreated and then treated concurrently with BFA and $^{35}$S met/cys (in the presence of TM in the originally treated group) as described above followed by a 2 hour chase. Crude cell extracts were immunoprecipitated (IP) either with polyclonal antisera prepared against AMK membrane preparations described above or with pan-anti heat shock Mab N6. Autoradiographs of the SDS-PAGE separated IP with either antibody of total cellular protein shows the presence of a $^{35}$S labeled 46.5 KDa protein in CIK resistant untreated lymphoblastoid cells which was absent in the TM treated cells.

EC targets were cultured in the presence of no BFA, the protective dose of 10 $\mu$m BFA, or the non-protective dose of 30 $\mu$M BFA concurrently with $^{35}$S met/cys as described above. Samples were processed as described for the AMK cells. EC targets cultured in the presence of BFA also contained a $^{35}$S labeled 46.5 KDa protein. This protein was not detectable in IP from EC cultured without BFA or with the non-protective dose of 30 $\mu$M BFA (see Figure 5).

This 46.5 KDa protein was identified as Hsp47 in a Western analysis using the Mab SPA-470 antibody. $^{35}$S labeled HUVEC extracts prepared from BFA treated cells described above were immunoprecipitated as described using the N6 pan-heat shock Mab. Precipitates were run on a 12% SDS-PAGE, transferred to NC membrane, blocked and reacted with the SPA-470 antibody using techniques well known to those of skill in the art. The presence of the SPA-470 antibody bound to the 46.5 KDa protein and the presence of the N6 heavy and light chains were detected using goat-anti mouse horseradish peroxidase staining assay(ECL).

Biotinoylation of live adherant EC with water soluble, lipid insoluble Biotin analog NHS-LC-biotin performed as described above followed by IP of cell extracts using either the anti-AMK polyclonal antibody or N6 Mab and subsequent SDS-PAGE, transfer to NC membrane and detection using a streptavidin-Alkaline phospahtase system showed that the Hsp47 protein induced by BFA treatment and a minor protein of 27 KDa are localized on the cell surface of EC targets.

**Example 7**

Purification and Analysis of Hsp47 Proteins

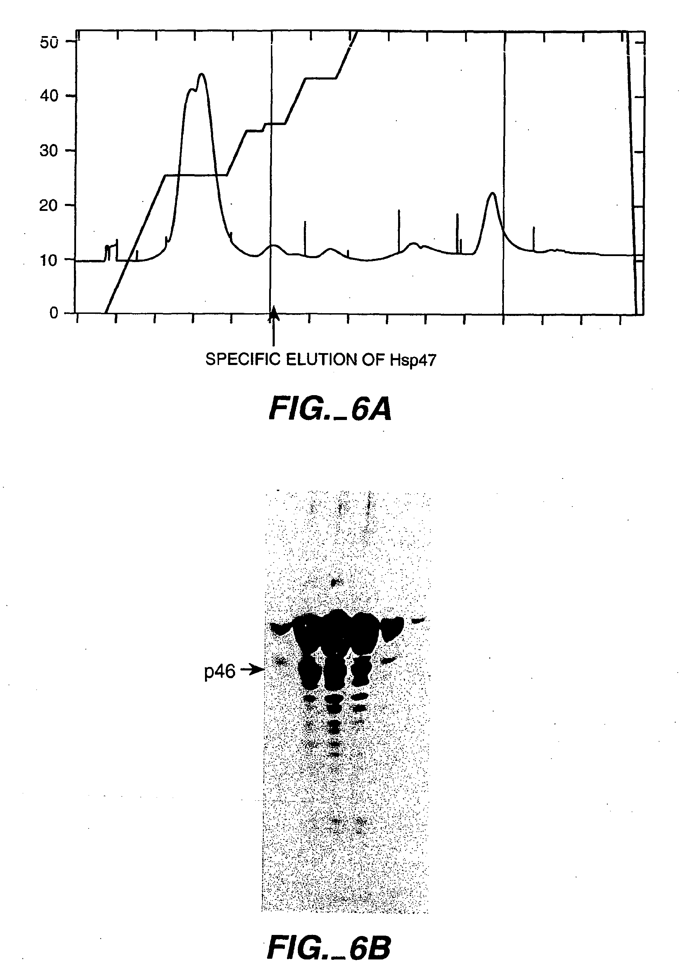

Native and recombinant protein purification was performed by subjecting protein crude extracts to anion-exchange FPLC chromatography on Q-sepharose® at pH 7.4, and pH 6.8, eluting separated fractions by increasing ionic strength and subsequent size exclusion FPLC chromatography on Superose 12 separating fractions with increasing elution volumes Ve, controlled by a UV-M monitor 214, 260 and 280nm and recorded in analogue mode (all equipment and resins: Pharmacia, Uppsala, Sweden) (see Figure 6). Protein amounts were quantitated on-line by integration of the area under the recorded peak of absorbance (AuP) and by Bradford assay of sampled fractions. Qualitative analysis was performed by SDS-PAGE and subsequent Coomassie and silver staining, as well as membrane transfer and western blot, when specific antibodies were available.

Figure 6A is a $UV_{260nm}$ profile of native Hsp47 purification from EC on Q-sepharose ff anion exchange resin at pH 8.9 175 flasks of confluent HUVEC were induced with 10 $\mu$M BFA as described. In the first step of ion exchange purification, proteins were separated with the same resin at pH 7.4 and hsp47 containing fractions pooled. Subsequently the proteins were diluted five fold in low salt buffer at pH 8.9 to yield buffer A (75 mM Tris•Cl pH 8.9, 175 mM NaCl, 2 mM DTT, 0.5 mM EDTA, 2 mM PMSF), and loaded on the ion exchange column. Bound proteins were subjected to a linear salt gradient with buffer B = A+100 mM NaCl. Separation of individual protein peaks was enhanced by manually switched step salt concentration plateaus, based upon OD extinction.

Figure 6B is a 12% SDS-PAGE analysis with silver staining of the collected p46.5 containing fractions. Those were pooled and concentrated by YD10 membrane to 1.0 ml.

Figure 6C is a 12% SDS-PAGE analysis with silver staining of the collected p46.5 containing fraction after size exclusion FPLC chromatography on Superose 12. Yield: 240$\mu$g.

Affinity FPLC chromatography was performed by loading protein extracts of wt and BFA treated EC onto gelatin-sepharose (Pharmacia, Uppsala, Sweden) matrix with help of a peristaltic pump and incubating the extract for 30 minutes at 0-4 °C. Unbound and unspecifically bound proteins were removed by washing with 7 column volumes of high salt buffer followed by original protein extraction buffer until the UV260nm detection at AU 0.02 returned to baseline. pH specific elution was carried out with PBS buffer at pH 5.5, alternative eluents included 100 mM ATP in PBS and 100 $\mu$M RGD peptide in PBS, both at pH 7.4.

Immunoaffinity FPLC chromatography was carried out in a similar manner, with the protein extract loaded onto a specific Mab column: 10 mg of purified Mab N6 was coupled via a covalent thioether bond with a 13 atom spacer arm to activated, crosslinked agarose via the Sulfolink (Pierce, Rockford, IL) protocol. Unspecifically bound proteins were removed from the columns as above, specifically immuno recognized proteins were eluted via pH 2.5 elution in 50 mM Na*citrate buffer.

Hsp47 may also be gelatin-affinity purification. 175 flasks of confluent HUVEC were induced with 10 $\mu$M BFA for 6 h at 37°C, 5% $Co_2$, in F12K medium supplemented with 20% FCS and standard EC culture supplements. Subsequently, adherent EC were harvested by scraping in detergent buffer (75 mM Tris•Cl, 175 mM NaCl, 2 mM DTT, 0.5 mM EDTA, 0.5% Tx-100, 2 mM PMSF), lysed by sonication and the extract cleared from cell debris by centrifugation at 15,000 rpm in a refrigerated eppendorf centrifuge. The cleared extract was loaded onto a 5 ml covalently coupled gelatin 4B Sepharose column via peristaltic pump at 0.5 ml $min^{-1}$. Unbound proteins were discarded. The column was then washed with detergent free buffer (as above, w/o Tx-100), low salt buffer (no NaCl) and high salt buffer (plus 500 mM NaCl) under $UV_{260nm}$ control until baseline readings were achieved. Specific elution of Hsp47 was achieved by lowering the pH of the detergent free buffer to pH 4.8. Collected eluate was immediately back-titrated to pH 7.4. The column was immediately regenerated and washed with pH 7.4 buffer.

Figure 2 shows the results of incubation of EC with gelatin-affinity purified Hsp47 for 30 minutes at 21°C, prior

to $^{51}$Cr release CIK cytotoxicity assay. Excess non-bound Hsp47 protein was removed from the wells by three washes prior to the addition of CIK effector cells. As a control, untreated BFA induced HUVEC targets were compared to the protein treatment group. Human Hsp47, the protein of 46.5 KDa purified with pH specific elution from gelatin matrix, protects EC.

**Example 8**

Hsp47 Peptides

Peptide Synthesis

A hydrophilic 9 mer peptide of the sequence AVLSAEQLR which corresponds to the region of shared homology of Hsp47 with HLA-A2 was synthesized by the Stanford PAN facility on a BioApplied® peptide synthesizer, HPLC purified and verified by amino acid sequencing. Pure peptide was lyophilized and stored at -20 °C. The peptide was soluble in aqueous solution and used in PBS and RPMI 1640 at indicated concentrations.

Amino acid sequencing of purified native p46 and proteolytic fragments derived from it was performed by A. Willis (Immuno Chemistry Unit of the MRC, Oxford University, Oxford, UK).

**Example 9**

In-vivo Analysis

SCID/hu Skin Graft-Melanoma-CIK Tumor Purging Model

Female 6 week old balbc/SCID mice were irradiated at 500 Rads at the day prior to skin grafting. Human full thickness foreskin grafts were prepared by aseptically exposing their subcutaneous vascular beds, and trimming their edges to yield a 20x30 mm graft. Foreskins yielding only smaller grafts or older than 24 hours of shelf age were rejected. Anesthetized animals were prepared in a 37 °C heated operative environment by aseptically denuding matching areas on their flanks. The full-thickness skin grafts were placed with interrupted absorbable suture, aseptically bandaged and the grafted animals housed separately until full engraftment was evident. After engraftment, $1x10^5$ melanoma WN9 or 1205 LU cells in PBS were injected intradermally into a single site of the human full-thickness skin of triplicate animals and tumor growth monitored by observation and palpation. Animals with evident nodular tumor burden exceeding a diameter of 5 mm were injected intraperitoneally with $1x10^7$ CIK cells (suspended in RPMI without FCS or IL-2) of proven lytic activity in same day $^{51}$Cr release cytotoxicity assay at CIK culture day d14-21. Animals were sacrificed at 0, 24, 48 and 72 hours following CIK injection, internal organs and the human skin/melanoma graft fixed separately and analyzed by light microscopy ofH&E and immuno-peroxidase anti-human CD3, human PE-CAM and human Kir antigens stained tissue sections. IP injected CIK cells selectively infiltrated the vascular beds of the melanoma tumor, which was confirmed by IPOX histologic immunodetection using anti-human PE-CAM Mab. Excluding a mild infiltrate in the portal area of the mouse liver, vasculature and skin tissue of the normal human skin graft or the murine host were not infiltrated by CIK *in vivo.*

SCID/hu HeLa Luciferase Hsp47 Secretion CIK Tumor Purging Live Imaging Model

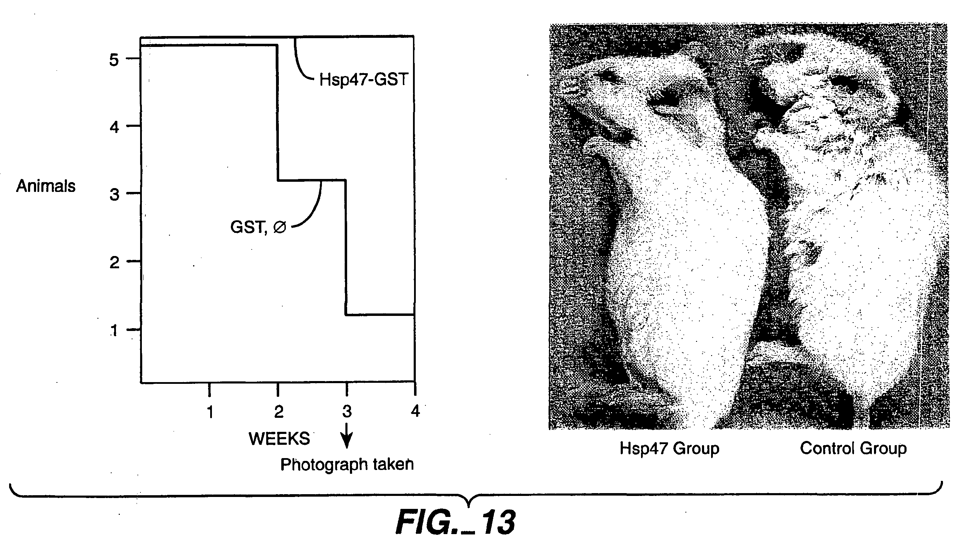

Female 6 week old balbc/SCID were irradiated at 500 Rads at the day prior to intraperitoneal injection of $2x10^4$ HeLa$_{luciferase}$ cervical carcinoma cells capable of imagable in-vivo luminescence. The CIK treatment group were injected with CIK at a 20:1 E:T ratio ($1x10^6$ cells) via a tail vein injection at the time of the HeLa cell challenge. Those HeLa$_{luciferase}$ cells were transiently transformed 24 hours prior to injection via lipofectamine (GIBCO/BRL, NY) protocol with a vector constituently expressing full-length huHsp47 directed to secretion under immediate early CMV promotor control or respective irrelevant control vectors. Transfection efficiency was monitored via co-transfection of eGFP and the Hsp47 construct with subsequent fluorescence laser microscopy analysis of transformed HeLaluc monolayers with a Axiophot (Zeiss, Jena, Germany) fluorescence microscope described above. Injected animals were monitored at 0, 2 and 7 days for *in-vivo* chemo-luminescence of remaining HeLa$_{luciferase}$ post CIK challenge with a quantitative digital imaging process in collaboration with Dr. C. Contag (see Figure 13). Animals receiving the CIK injection demonstrated a dramatic decrease in live, light emmitting tumor burden one week after CIK purging. Animals which did not receive the CIK cells demonstrated widespread IP growth of HeLa cells. The CIK cells demonstrated a high and rapid solid tumor purging potential.

Murine GvHDModel

Male Balb/c mice were twice irradiated with 500 Rads prior to transplantation. Bone marrow (BM) and spleen derived stem cells were obtained from B10D2 (H-2$^d$ background) mice. 2.5x10$^6$ BM and 1x10$^6$ spleen cells were pooled as a graft source for each recipient male Balb/c animal. This non-enriched stem cell population of cells were either suspended in RPMI containing 300 µg Hsp47-Gst protein in 300 µl total volume, or, in buffer containing 100 µg of Gst control protein alone. Cells and protein were given in a single injection on day 0. No other immunosuppressive treatments were given. Animals were monitored for 4 weeks after the bone marrow transplant. All 5 animals receiving the Hsp-Gst protein treatment were protected from the onset of GvHD, fur ruffling, weight loss and death. Animals receiving only the Gst protein all exhibited signs of GvHD, fur ruffling and weight loss, and 4 of the 5 animals died (see Figure 15).

**Example 10**

FACS Analysis

This Example discloses the use of modified Hsp47 to identify and separate immune cell subsets.

A variety of immune cells respond to Hsp47 and do not attack endothelial cells. In order to identify such cells and to enrich adoptive immunotherapeutic populations from these non-endothelial reactive cells in the presence of Hsp47, fluorescent tagged Hsp47 can be used in a standard cell sorting apparatus to identify and separate such cells. Such use can also serve as a diagnostic tool for immune cell subset analysis in various disease states.

eGFP-tagged Hsp47 FACS probe was generated through additional genetic engineering (Figure 8A). This enabled searching for both potential huHsp47 receptors and for CIK subsets expressing such Hsp47 binding proteins. FACS analysis of mature d$_{21}$ CIK with eGFP-tagged HuHsp47 as FACS probe for the FITC channel, with CE56-PE co-staining, is based on differences in the Hsp47 binding abiligy of the analyzed CIK. The eGFP tagged Hsp47 probe stained a quarter of mature CIK (26%).

The foregoing description details specific methods which can be employed to practice the present invention. Having detailed such specific methods, those skilled in the art will well enough know how to devise alternative reliable methods at arriving at the same information in using the fruits of the present invention. Thus, however, detailed the foregoing may appear in text, it should not be construed as limiting the overall scope thereof; rather, the ambit of the present invention is to be determined only by the lawful construction of the appended claims.

**Claims**

1. A pharmaceutical composition comprising HuHsp47 polypeptide.

2. Use of a HuHsp47-related immunoprotective polypeptide for the preparation of a pharmaceutical composition for the prevention and/or treatment of diseases related to immune-system mediated cell damage.

3. Use of a huHsp47-related immunoprotective polypeptide according to claim 2, wherein said immune-system mediated damage is caused by an autoimmune disease, graft vs. host disease or host vs. graft disease.

4. A polypeptide with immunoprotective activity selected from the following:

   a) huHsp47
   b) the amino acid sequence shown in Fig. 1
   c) an amino acid sequence with immunoprotective activity having at least about 70% amino acid sequence identity with the amino acid sequence of a) or b)
   d) an amino acid sequence with immunoprotective activity encoded by a nucleic acid that hybridizes to a nucleic acid that encodes the amino acid sequence of a) or b)
   e) a fragment of a), b), c) or d) with immunoprotective activity
   f) a variant of a), b), c) or d) with immunoprotective activity.

**5.** An isolated nucleic acid encoding a polypeptide with immunoprotective activity selected from the group consisting of:

a) a nucleic acid encoding huHsp47
b) the nucleic acid sequence shown in Fig. 1
c) a nucleic acid sequence with immunoprotective activity having at least about 70% nucleic acid sequence identity with the amino acid sequence of a)
d) a nucleic acid sequence with immunoprotective activity that hybridizes to a nucleic acid that encodes the amino acid sequence shown in Fig. 1
e) a fragment of a), b), c) or d) encoding a polypeptide with immunoprotective activity
f) a variant of a), b), c) or d) encoding a polypeptide with immunoprotective activity.

**6.** A vector comprising nucleic acid encoding human Hsp47 polypeptide operably linked to control sequences recognized by a host cell transformed with the vector.

**7.** A host cell comprising the vector of Claim 6.

**8.** A method for reducing immune-system mediated damage to tissue, organs or cells comprising contacting the tissue, organs or cells with a HuHsp47-related immunoprotective polypeptide wherein said method is in vitro or ex vivo.

**9.** Use of a polypeptide according to claim 4 or of a nucleic acid according to claim 5 for the preparation of a pharmaceutical composition for the prevention and/or treatment of diseases related to immune-system mediated cell damage.

**10.** Use of a polypeptide or nucleic acid according to claim 9, wherein said immune-system mediated damage is caused by an autoimmune disease, graft vs. host disease or host vs. graft disease.

**11.** Use of a polypeptide according to claim 4 or of a nucleic acid according to claim 5 for the preparation of a composition for an adoptive immune therapy to treat cancer wherein said composition is designed to be introduced into a patient in conjunction with T-lymphocytes obtained by culturing T-lymphocytes from a donor under conditions wherein specific subsets of said T-lymphocytes are expanded.

**12.** A method for reducing immune-system mediated damage to tissue, organs or cells comprising contacting a tissue, organ or cell with an expressible nucleic acid encoding a polypeptide according to claim 4 or with a nucleic acid according to claim 5 wherein said method is in vitro or ex vivo.

**13.** An **fax** Hsp47-related immunoprotective polypeptide as defined in any one of claims above in combination with a cell, tissue or organ.

**Patentansprüche**

**1.** Eine pharmazeutische Zusammensetzung mit HuHsp47 Polypeptid.

**2.** Verwendung eines HuHsp47 verwandten immunprotektiven Polypeptids zur Herstellung einer pharmazeutischen Zusammensetzung zur Prävention und/oder Behandlung von Zellschädigungen, die von Wirkungen bzw. Krankheiten des Immunsystems hervorgerufen werden.

**3.** Verwendung eines HuHsp47 verwandten immunprotektiven Polypeptids gemäß Anspruch 2, bei dem die Immunsystem-bedingte Zellschädigung durch eine Autoimmunkrankheit, Graft vs. Host Reaktion oder Host vs. Graft Reaktion ausgelöst wird.

**4.** Ein Polypeptid mit immunprotektiver Aktivität ausgewählt aus:

a) HuHsp47
b) die Aminosäuresequenz der Fig. 1
c) eine Aminosäuresequenz mit immunprotektiver Aktivität mit mindestens 70% Sequenzidentität mit der Aminosäuresequenz von a) oder b)
d) eine Aminosäuresequenz mit immunprotektiver Aktivität kodiert durch eine Nukleinsäure, die hybridisiert mit einer Nukleinsäure, die Aminosäuresequenz a) oder b) kodiert

e) ein Fragment von a), b), c) oder d) mit immunprotektiver Aktivität

f) eine Variante von a), b), c) oder d) mit immunprotektiver Aktivität

5. Eine isolierte Nukleinsäure kodierend für ein Polypeptid mit immunprotektiver Aktivität ausgewählt aus der Gruppe bestehend aus:

a) Ein Nukleinsäure kodierend für huHsp47

b) die Nukleinsäure der Fig. 1

c) eine Nukleinsäure mit immunprotektiver Aktivität mit mindestens 70% Sequenzidentität mit der Nukleinsäure von a)

d) eine Nukleinsäure mit immunprotektiver Aktivität, die hybridisiert mit einer Nukleinsäure die Aminosäuresequenz der Fig. 1 kodiert

e) ein Fragment von a), b), c) oder d) kodierend für ein Polypeptid mit immunprotektiver Aktivität

f) eine Variante von a), b), c) oder d) kodierend für ein Polypeptid mit immunprotektiver Aktivität.

6. Ein Vektor mit einer Nukleinsäure kodierend für humanes Hsp47 Polypeptid, funktionell an Kontrollsequenzen gekoppelt, die durch eine mit dem Vektor transfizierte Wirtszelle erkannt werden.

7. Eine Wirtszelle mit dem Vektor des Anspruchs 6.

8. Ein Verfahren zur Verminderung Immunsystem-bedingter Schädigung von Gewebe, Organen oder Zellen mit den Schritten eines Kontaktierens des Gewebes, Organs oder der Zellen mit einem HuHsp47 verwandten immunprotektiven Polypeptid bei dem das Verfahren in vitro oder in vivo durchgeführt wird.

9. Verwendung eines immunprotektiven Polypeptids nach Anspruch 4 oder einer Nukleinsäure nach Anspruch 5 zur Herstellung einer pharmazeutischen Zusammensetzung zur Prävention und/oder Behandlung von Zellschädigungen die von Wirkungen bzw. Krankheiten bzw. Immunsystems hervorgerufen werden.

10. Verwendung eines Polypeptids nach Anspruch 9, wobei die Immunsystem-bedingte Zellschädigung von einer Autoimmunkrankheit, einer Graft vs. Host Reaktion oder einer Host vs. Graft Reaktion hervorgerufen werden.

11. Verwendung eines immunprotektiven Polypeptids nach Anspruch 4 oder einer Nukleinsäure nach Anspruch 5 zur Herstellung einer Zusammensetzung für eine adoptive Immuntherapie zur Behandlung von Krebs, wobei die Zusammensetzung derart ausgebildet ist, dass sie einem Patienten zusammen mit T-Lymphozyten aus einer T-Lymphozytenkultur eines Donors verabreicht werden kann, und bei den T-Lymphozyten bestimmte spezifische Untergruppen expandiert werden.

12. Verfahren zur Verminderung Immunsystem-bedingter Schädigung von Gewebe, Organen oder Zellen mit den Schritten Herstellen eines Kontakts zu einem Gewebe, Organ oder Zellen mit einer expressiblen Nukleinsäure kodierend für eine Polypeptid nach Anspruch 4 oder einer Nukleinsäure nach Anspruch 5, wobei das Verfahren in vitro oder ex vivo durchgeführt wird.

13. Ein HuHsp47 verwandtes immunprotektives Polypeptid nach einem der vorhergehenden Ansprüche in Kombination mit einer Zelle, einem Gewebe oder einem Organ.

**Revendications**

1. Composition pharmaceutique comprenant un polypeptide HuHsp47.

2. Utilisation d'un polypeptide immunoprotecteur apparenté à HuHsp47 pour la préparation d'une composition pharmaceutique pour la prévention et/ou le traitement de maladies liées à des lésions de cellules médiées ou induites par le système immunitaire.

3. Utilisation d'un polypeptide immunoprotecteur apparenté à huHsp47 selon la revendication 2, dans laquelle lesdites lésions médiées par le système immunitaire sont causées par une maladie auto-immune, une maladie du greffon contre l'hôte ou une maladie de l'hôte contre le greffon.

**4.** Polypeptide avec une activité immunoprotectrice sélectionné parmi les éléments suivants :

    a) huHsp47

    b) la séquence d'acides aminés montrée en Figure 1

    c) une séquence d'acides aminés avec une activité immunoprotectrice dont la séquence d'acides aminés est identique à environ 70 % au moins à la séquence d'acides aminés de a) ou de b)

    d) une séquence d'acides aminés avec une activité immunoprotectrice codée par un acide nucléique qui s'hybride à un acide nucléique qui code la séquence d'acides aminés de a) ou de b)

    e) un fragment de a), b), c) ou d) avec une activité immunoprotectrice

    f) une variante de a), b), c) ou d) avec une activité immunoprotectrice.

**5.** Acide nucléique isolé codant un polypeptide avec une activité immunoprotectrice sélectionné parmi le groupe constitué de :

    a) un acide nucléique codant huHsp47

    b) la séquence d'acides nucléiques montrée en Figure 1

    c) une séquence d'acides nucléiques avec une activité immunoprotectrice dont la séquence d'acides nucléiques est identique à environ 70 % au moins à la séquence d'acides aminés de a)

    d) une séquence d'acides nucléiques avec une activité immunoprotectrice qui s'hybride à un acide nucléique qui code la séquence d'acides aminés montrée en Figure 1

    e) un fragment de a), b), c) ou d) avec un polypeptide avec une activité immunoprotectrice

    f) une variante de a), b), c) ou d) codant un polypeptide avec une activité immunoprotectrice.

**6.** Vecteur comprenant un acide nucléique codant un polypeptide Hsp47 humain lié de manière opérationnelle à des séquences de contrôle reconnues par une cellule hôte transformée avec le vecteur.

**7.** Cellule hôte comprenant le vecteur selon la revendication 6.

**8.** Procédé pour réduire les lésions médiées par le système immunitaire sur les tissus, les organes ou les cellules comprenant de mettre en contact avec les tissus, les organes ou les cellules avec un polypeptide immunoprotecteur apparenté à HuHsp47 dans lequel ledit procédé est un procédé in vitro ou ex vivo.

**9.** Utilisation d'un polypeptide selon la revendication 4 ou d'un acide nucléique selon la revendication 5 pour la préparation d'une composition pharmaceutique pour la prévention et/ou le traitement des maladies liées à des lésions des cellules médiées ou induites par le système immunitaire.

**10.** Utilisation d'un polypeptide ou d'un acide nucléique selon la revendication 9, dans laquelle lesdites lésions médiées ou induites par le système immunitaire sont causées par une maladie auto-immune, une maladie du greffon contre l'hôte ou une maladie de l'hôte contre le greffon.

**11.** Utilisation d'un polypeptide selon la revendication 4 ou d'un acide nucléique selon la revendication 5 pour la préparation d'une composition pour une thérapie adoptive immunitaire afin de traiter le cancer dans laquelle ladite composition est réalisée pour être induite chez le patient en conjonction avec des lymphocytes T obtenus en cultivant des lymphocytes T d'un donneur dans des conditions dans lesquelles des sous-ensembles desdits lymphocytes T subissent une expansion.

**12.** Procédé pour réduire les lésions médiées par le système immunitaire des tissus, des organes ou des cellules comprenant de mettre en contact un tissu, un organe ou une cellule avec un acide nucléique exprimable codant un polypeptide selon la revendication 4 ou avec un acide nucléique selon la revendication 5 dans lequel ledit procédé est un procédé in vitro ou ex vivo.

**13.** Polypeptide immunoprotecteur apparenté à HuHsp47 tel que défini dans l'une quelconque des revendications ci-dessus en association avec une cellule, un tissu ou un organe.

<pre>
              Signal Peptide                        |Mature
    M   R   S   L   L   L   G   T   L   C   L   L   A   V   A   L   A | A   E   V   K   K
  ATGCGCTCTCTCCTTCTGGGCACCTTATGCCTCCTGGCTGTGGCCCTGGCAGCCGAGGTGAAGAAA
  TACGCGAGAGAGGAAGACCCGTGGAATACGGAGGACCGACACCGGGACCGTCGGCTCCACTTCTTT
    P   V   E   A   A   A   P   G   T   A   E   K   L   S   S   K   A   T   T   L   A   E
  CCTGTAGAGGCCGCAGCCCCTGGTACTGCGGAGAAGCTGAGTTCCAAGGCGACCACACTGGCAGAG
  GGACATCTCCGGCGTCGGGGACCATGACGCCTCTTCGACTCAAGGTTCCGCTGGTGTGACCGTCTC
    P   S   T   G   L   A   F   S   L   Y   Q   A   M   A   K   D   Q   A   V   E   N   I
  CCCAGCACAGGCCTGGCCTTCAGCCTGTATCAGGCAATGGCCAAGGACCAGGCAGTGGAGAACATC
  GGGTCGTGTCCGGACCGGAAGTCGGACATAGTCCGTTACCGGTTCCTGGTCCGTCACCTCTTGTAG
    L   V   S   P   V   V   V   A   S   S   L   G   L   V   S   L   G   G   K   A   T   T
  CTGGTGTCACCCGTGGTGGTGGCCTCGTCGCTGGGTCTCGTGTCGCTGGGCGGCAAGGCGACCACG
  GACCACAGTGGGCACCACCACCGGAGCAGCGACCCAGAGCACAGCGACCCGCCGTTCCGCTGGTGC
    A   S   Q   A   K   A   V   L   S   A   E   Q   L   R   D   E   E   V   H   A   G   L
  GCGTCGCAGGCCAAGGCAGTGCTGAGCGCCGAGCAGCTGCGCGACGAGGAGGTGCACGCCGGCCTG
  CGCAGCGTCCGGTTCCGTCACGACTCGCGGCTCGTCGACGCGCTGCTCCTCCACGTGCGGCCGGAC
    G   E   L   L   R   S   L   S   N   S   T   A   R   N   V   T   W   K   L   G   S   R
  GGTGAGCTGCTGCGCTCACTCAGCAACTCGACGGGCGCGCAACGTGACCTGGAAGCTGGGCAGCCGA
  CCACTCGACGACGCGAGTGAGTCGTTGAGCTGCCGCGCGTTGCACTGGACCTTCGACCCGTCGGCT
    L   Y   G   P   S   S   V   S   F   A   D   D   F   V   R   S   S   K   Q   H   Y   N
  CTGTACGGACCCAGCTCAGTGAGCTTCGCTGATGACTTCGTGCGCAGCAGCAAGCAGCACTACAAC
  GACATGCCTGGGTCGAGTCACTCGAAGCGACTACTGAAGCACGCGTCGTCGTTCGTCGTGATGTTG
    C   E   H   S   K   I   N   F   P   D   K   R   S   A   L   Q   S   I   N   E   W   A
  TGCGAGCACTCCAAGATCAACTTCCCGGACAAGCGCAGCGCGCTGCAGTCCATCAACGAGTGGGCC
  ACGCTCGTGAGGTTCTAGTTGAAGGGCCTGTTCGCGTCGCGCGACGTCAGGTAGTTGCTCACCCGG
    A   Q   T   T   D   G   K   L   P   E   V   T   K   D   V   E   R   T   D   G   A   L
  GCGCAGACCACCGACGGCAAGCTGCCCGAGGTCACCAAGGACGTGGAGCGCACGGACGGCGCCCTG
  CGCGTCTGGTGGCTGCCGTTCGACGGGCTCCAGTGGTTCCTGCACCTCGCGTGCCTGCCGCGGGAC
    L   V   N   A   M   F   F   K   P   H   W   D   E   K   F   H   H   K   M   V   D   N
  CTAGTCAACGCCATGTTCTTCAAGCCACACTGGGATGAGAAATTCCACCACAAGATGGTGGACAAC
  GATCAGTTGCGGTACAAGAAGTTCGGTGTGACCCTACTCTTTAAGGTGGTGTTCTACCACCTGTTG
    R   G   F   M   V   T   R   S   Y   T   V   G   V   T   M   M   H   R   T   G   L   Y
  CGTGGCTTCATGGTGACTCGGTCCTATACTGTGGGTGTTACGATGATGCACCGGACAGGCCTCTAC
  GCACCGAAGTACCACTGAGCCAGGATATGACACCCACAATGCTACTACGTGGCCTGTCCGGAGATG
    N   Y   Y   D   D   E   K   E   K   L   Q   L   V   E   M   P   L   A   H   K   L   S
  AACTACTACGACGACGAGAAGGAGAAGCTGCAGCTGGTGGAGATGCCCCTGGCTCACAAGCTCTCC
  TTGATGATGCTGCTGCTCTTCCTCTTCGACGTCGACCACCTCTACGGGGACCGAGTGTTCGAGAGG
    S   L   I   I   L   M   P   H   H   V   E   P   L   E   R   L   E   K   L   L   T   K
  AGCCTCATCATCCTCATGCCCCATCACGTGGAGCCTCTCGAGCGCCTTGAAAAGCTGCTAACCAAA
  TCGGAGTAGTAGGAGTACGGGGTAGTGCACCTCGGAGAGCTCGCGGAACTTTTCGACGATTGGTTT
    E   Q   L   K   I   W   M   G   K   M   Q   K   K   A   V   A   I   S   L   P   K   G
  GAGCAGCTGAAGATCTGGATGGGGAAGATGCAGAAGAAGGCTGTTGCCATCTCCTTGCCCAAGGGT
  CTCGTCGACTTCTAGACCTACCCCTTCTACGTCTTCTTCCGACAACGGTAGAGGAACGGGTTCCCA
    V   V   E   V   T   H   D   L   Q   K   H   L   A   G   L   G   L   T   E   A   I   D
  GTGGTGGAGGTGACCCATGACCTGCAGAAACACCTGGCTGGGCTGGGCCTGACTGAGGCCATTGAC
  CACCACCTCCACTGGGTACTGGACGTCTTTGTGGACCGACCCGACCCGGACTGACTCCGGTAACTG
    K   N   K   A   D   L   S   R   M   S   G   K   K   D   L   Y   L   A   S   V   F   H
  AAGAACAAGGCCGACTTATCACGCATGTCTGGCAAGAAGGATCTGTACCTGGCCAGTGTGTTCCAC
  TTCTTGTTCCGGCTGAATAGTGCGTACAGACCGTTCTTCCTAGACATGGACCGGTCACACAAGGTG
    A   T   A   F   E   L   D   T   D   G   N   P   F   D   Q   D   I   Y   G   R   E   E
  GCCACCGCCTTTGAGTTGGACACAGATGGCAACCCCTTTGACCAGGACATCTACGGGCGCGAGGAG
  CGGTGGCGGAAACTCAACCTGTGTCTACCGTTGGGGAAACTGGTCCTGTAGATGCCCGCGCTCCTC
    L   R   S   P   K   L   F   Y   A   D   H   P   F   I   F   L   V   R   D   T   Q   S
  CTGCGCAGCCCCAAGCTGTTCTACGCCGACCACCCCTTCATCTTCCTGGTGCGGGACACCCAAAGC
  GACGCGTCGGGGTTCGACAAGATGCGGCTGGTGGGGAAGTAGAAGGACCACGCCCTGTGGGTTTCG
    G   S   L   L   F   I   G   R   L   V   R   L   K   G   D   K   M   R   D   E   L   •
  GGCTCCCTGCTATTCATTGGGCGCCTGGTCCGGCTCAAGGGTGACAAGATGCGAGACGAGTTATAG
  CCGAGGGACGATAAGTAACCCGCGGACCAGGCCGAGTTCCCACTGTTCTACGCTCTGCTCAATATC
</pre>

## FIG._1

## Alignment of HSO47 with HLA/IL-12β Chains

| Molecule | Position | Amino Acid Sequence | Position |
|---|---|---|---|
| Human Hsp47 | 96 | AvlsAEQLR | 104 |
| Hu Colligin 2 | 95 | AVLSAEQLR | 103 |
| HLA-A2 A*0201 | 149 | AahvAEQLR | 157 |
| HLA-A2 A*0204 | 167 | AahvAEQLR | 175 |
| HLA-A2 A*0206 | 175 | AahvAEQLR | 183 |
| HLA-A2 A*0211 | 166 | AahvAEQLR | 174 |
| HLA-A10 alpha | 84 | AahvAEQLR | 92 |
| HLA-Aw 69 | 150 | AahvAEQLR | 158 |
| Rat Hsp47 | 94 | AVLSAEkLR | 102 |
| Mouse Hsp47 | 94 | AVLSAEkLR | 102 |
| Hamster Hsp47 | 94 | AVLSAEkLR | 102 |
| Chicken Hsp47 | 82 | AVLSAdkLn | 90 |
| Hu IL-12 β-chain | 150 | AtLSAErvR | 158 |
| mouseIL1 2β-chain | 150 | AtLSAErvR | 158 |
| cat IL-12 β-chain | 150 | AtLSAEkvR | 158 |
| cow IL12 β-chain | 150 | AlLSAEkvn | 158 |

Consensus HSP          AVLSA(d,e)(k,q)LR

Consensus HLA          A(v,a)(l,h)(a,v)AEQLR

Consensus IL-12 β-chain          A(v,l,t)LSAE(q,k,r)(l,v)R

Overall Consensus          A(v,l,a,t)(l,h)(a,v)A(d,e)(k,q,r)(l,v)R

## FIG._2

% SPECIFIC LYSIS

OCI-Ly 8, SU-DHL4

K 562

WM9

HU-VEC

**FIG._3A**

1:1    2:1    5:1    10:1    20:1    40:1

EFFECTOR: TARGET RATIO

% SPECIFIC LYSIS

HUV-EC, Freshly Confluent

HUV-EC, 5 Days Confluent

**FIG._3B**

1:1    2:1    5:1    10:1    20:1    40:1

EFFECTOR: TARGET RATIO

% SPECIFIC LYSIS

OCI-Ly8

HU-VEC

**FIG._3C**

2D10          5F7          2C10

CLONE

43

FIG._4

% SPECIFIC
LYSIS

$d_{21}$ CIK EFFECTOR: TARGET RATIO 20:1

FIG._7

% SPECIFIC
LYSIS

TREATMENT

FIG._5A          FIG._5B          FIG._5C

SPECIFIC ELUTION OF Hsp47

*FIG._6A*

p46 →

*FIG._6B*

**FIG._8A**

Plasmid name: huHsp47/pUC19
Plasmid size: 3910 bp
Constructed by: Ernest-G. Hope
Construction date: 1996
Comments/References: puc 19 based huHsp47 gene cassette derived from 2 partial
clones in pCR1000/pGEM via primers (A.5′-ACGTTTGGATCCAGGTGAAGA,3′-GTCCTTGGCCAT
B.5′ GCAATGGCCAAGGACCAGGCAGTGGAG,3′ATCTGAATTCCTATAACTCGTCTCGCA)

**FIG._8B**

FIG._9

FIG._10

FIG._11

FIG._12

Hsp47-GST

GST, Ø

Animals

5

4

3

2

1

1    2    3    4

WEEKS ↓

Photograph taken

Hsp47 Group    Control Group

*FIG._13*

**FIG._14A**

**FIG._14B**

N-term

C-term

RGD-Binding-    HLA-Consensus    Serine Pretease Inhibitor-    ER Signal

## FIG._15A

GFP* FACS Probe
Deletion 1
Deletion 2
Deletion 3

## FIG._15B

% SPECIFIC
LYSIS

$d_{21}$ CIK : EC Target Ratio of 20:1

## FIG._15C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 07233083 A
- WO 9521614 A, Satoh
- EP 0813871 A
- US 9919337 W
- US 09382088 B
- US 60097640 B

### Non-patent literature cited in the description

- **SULLIVAN et al.** *Blood,* 1989, vol. 73, 1720
- **WEIDEN et al.** *N. Engl. J. Med.,* 1979, vol. 300, 1068-1073
- **ANTIN.** *Blood,* 1993, vol. 82, 2273-2277
- **HAUCH et al.** *Blood,* 1990, vol. 75, 2250-2262
- **MARTIN et al.** *Blood,* 1985, vol. 66, 664-672
- **COLLINS et al.** *Clinical Oncology,* 1997, vol. 15, 433-444
- **KOLB et al.** *Blood,* 1995, vol. 86, 2041-2050
- **PORTER et al.** *N. Engl. J. Med.,* 1994, vol. 330, 100-106
- **GUIDOS et al.** *J. of Exp. Med.,* 1990, vol. 172, 835-845
- **WEISSMAN.** *Cell,* 1994, vol. 76, 207-218
- **HOGLUND et al.** *Immun. Rev.,* 1997, vol. 155, 11-28
- **BLAISE et al.** *Eur. Cytokine Netw.,* 1991, vol. 2, 121-129
- **FORTIS et al.** *Cancer Immunol. Immunother,* 1991, vol. 33, 128-132
- **LEE et al.** *J. Biol. Response Mod.,* 1988, vol. 7, 43-53
- **NALESNIK et al.** *Transplantation,* 1997, vol. 63, 1200-1205
- **ROSENBERG.** *J. Biol. Response Mod.,* 1984, vol. 3, 501-511
- **ROSENBERG et al.** *New England J. of Med.,* 1987, vol. 316, 889-897
- **ROSENBERG et al.** *J. of Exp. Med.,* 1985, vol. 161, 1169-1188
- **TEICHMANN et al.** *Leuk. Res.,* 1992, vol. 16, 287-98
- **SIEGEL et al.** *J. of Clin. Oncology,* 1991, vol. 9, 694-704
- **GLAUSER et al.** *Am. J. Med. Sci.,* 1988, vol. 296, 406-412
- **KOTASEK et al.** *Cancer Res.,* 1988, vol. 48, 5528-5532
- **KOZENY et al.** *J. Clin. Oncology,* 1988, vol. 6, 1170-1176
- **ROSENBERG et al.** *Ann. Surg.,* vol. 210, 474-484
- **ROSENBERG et al.** *New. Engl. J. of Med.,* 1988, vol. 319, 1676-1680
- **ROSENBERG et al.** *J. Nat. Canc. Inst.,* 1994, vol. 86, 1159-1166
- **LU et al.** *J. Immunol.,* 1994, vol. 153, 1687-1696
- **SCHMIDT-WOLF et al.** *Ann. Hematol.,* 1997, vol. 74, 51-56
- **SCHMIDT-WOLF et al.** *Exp. Hematol.,* 1993, vol. 21, 1673-1679
- **BLAHETA et al.** *Immunology,* 1998, vol. 94, 213-220
- **FINNEGAN et al.** *Cancer,* 1998, vol. 82, 186-199
- **UTOGUCHI et al.** *Inflammation,* 1997, vol. 21, 223-233
- **HOPPE ; NEGRIN.** Blood. *American Society of Hematology, Thirty-Ninth Annual Meeting,* 05 December 1997
- **JIANG et al.** *Immunology,* 1996, vol. 87 (3), 481-486
- **WADA et al.** *Jpn. J. Cancer Res.,* 1993, vol. 84 (8), 906-913
- **KRENSKY et al.** *Exp. Opin. Invest. Drugs,* 1996, vol. 5, 809
- *The Journal of Cell Biology,* April 1996, vol. 133 (2), 469-483
- **VERRICO et al.** *British Journal of Cancer,* 1997, vol. 76 (6), 719-724
- **ALTSCHUL et al.** *Methods in Enzymology,* 1996, vol. 266, 460-480, http://blast.wustl/edu/blast/README.html
- **GUSTIN et al.** *Biotechniques,* 1993, vol. 14, 22
- **BARANY.** *Gene,* 1985, vol. 37, 111-123
- **COLICELLI et al.** *Mol Gen Genet,* 1985, vol. 199, 537-539
- **PRENTKI et al.** *Gene,* 1984, vol. 29, 303-313
- **LOPEZ et al.** *Faseb. J.,* 1995, vol. 9, A1024
- **MEHTA et al.** *Blood,* 1995, vol. 86, 3493-3499

- **SHIELDS et al.** *Tissue Antigens,* 1998, vol. 51, 567-570
- **HOYLE et al.** *Blood,* 1998, vol. 92, 3318-3327
- **SCHMIDT-WOLF.** *Cellular Immunology,* 1996, vol. 169, 85-90
- **WULFING et al.** *Science,* 1998, vol. 282, 2266-2269
- **WULFING et al.** *Proc. Natl. Acad. Sci USA,* 1998, vol. 95, 6302-6307
- **LAWRENCE et al.** *Eur. J. Immunol.,* 1995, vol. 25, 1025-1031
- **OPPENHEIMER-MARKS et al.** *J. of Immunology,* 1990, vol. 145, 140-148
- **ROSENMAN et al.** *J. of Leukocyte Biol.,* 1993, vol. 53, 1-10
- **KOZENY et al.** *J. Clin. Oncol.,* 1988, vol. 6, 1170-1176
- **KATAOKA et al.** *J. Immunol.,* 1996, vol. 156, 3678-3686
- **SCHWARTZ.** *Curr. Opin. Immunol.,* 1997, vol. 9, 351-357
- **MADRENAS et al.** *Proc. Natl. Acad. Sci. USA,* 1996, vol. 93, 9736-9741
- **FINK et al.** *J. Immunol.,* 1994, vol. 152, 4270-4281
- **ALTERS et al.** *Transplantation,* 1993, vol. 56, 633-638
- **HOGLUND et al.** *Immunological Reviews,* 1997, vol. 155, 11-28
- **KARPUS et al.** *Int. Immunol.,* 1994, vol. 6, 721-730
- **KARRE.** *Semin. Cancer Biol.,* 1991, vol. 2, 295-309
- **LJUNGGREN et al.** *Immunol. Today,* 1990, vol. 11, 237-244
- **MADRENAS et al.** *Proc. Natl. Acad Sci. USA,* 1996, vol. 93, 9736-9741
- **NOSSAL.** *Ann. N. Y. Acad. Sci.,* 1993, vol. 690, 34-41
- **ROTHENBERG.** *Adv. Immunol.,* 1992, vol. 51, 85-214
- **LIPPINCOTT-SCHWARTZ et al.** *Cell,* 1989, vol. 56, 801-813
- **FERREIRA et al.** *Arch. Virol.,* 1994, vol. 138, 273-285
- **FERREIRA et al.** *J. Cell Biochem.,* 1994, vol. 56, 518-526
- **FERREIRA et al.** *Connect Tissue Res.,* 1996, vol. 33, 265-273
- **SMITH et al.** *J. Biol. Chem.,* 1995, vol. 270, 18323-18328
- **LLEWELLYN et al.** *Biochemical and Biophysical Research Communications,* 1997, vol. 240, 36-40
- **HU et al.** *J. Cell Biochem.,* 1995, vol. 59, 350-367
- **HU et al.** *J. Cell Biochem.,* 1995, vol. 59, 214-234
- **YAMAMURA et al.** *Biochem Biophys. Res. Comm.,* 1998, vol. 244, 68-74
- **HIRAYOSHI et al.** *Mol. Cell Biol.,* 1991, vol. 11, 4036-4044
- **JAIN et al.** *Biochem J.,* 1994, vol. 304, 61-68
- **NANDAN et al.** *Biochem Cell Biol.,* 1990, vol. 68, 1057-1061
- **VAILLANCOURT et al.** *Biochem J.,* 1991, vol. 274, 793-798
- **CLARKE et al.** *Biochem Biophys Acta,* 1992, vol. 1129, 246-248
- **HUGHES et al.** *Eur. J. Biochem.,* 1987, vol. 163, 57-65
- **CLARKE et al.** *J. Cell Biol.,* 1993, vol. 121, 193-199
- **HIRAYOSHI et al.** *Mol Cell Biol,* 1991, vol. 11, 4036-4044
- **DAVIDS et al.** *Bioorganic Chemistry,* 1995, vol. 23, 437-438
- **KARRE.** *Semin Cancer Biol,* 1991, vol. 2, 295-309
- **KARRE et al.** *Nature,* 1986, vol. 319, 675-678
- **LJUNGGREN et al.** *Immunol Today,* 1990, vol. 11, 237-244
- **HÖGLUND et al.** *Immunological Reviews,* 1997, vol. 155, 11-28
- **BREWER et al.** *Embo J.,* 1997, vol. 16, 7207-7216
- **NAKAI et al.** *Biochem Biophys Res Comm,* 1989, vol. 164, 259-264
- **HEBERT et al.** *J. Cell Biochem,* 1999, vol. 73, 248-258
- **LEVY et al.** *Annu Rev Immunol,* 1998, vol. 16, 89-109
- **LAGAUDRIERE-GESBERT et al.** *J. Immunol,* 1997, vol. 158, 2790-2797
- **KASAI et al.** *Cell Tissue Res,* 1995, vol. 281, 135-141
- **SAUK et al.** *Biochem Biophys Res Comm,* 1990, vol. 172, 135-142
- **CLARKE et al.** *J. Cell Biol,* 1993, vol. 121, 193-199
- **MORINO et al.** *In Vivo,* 1994, vol. 8, 285-288
- **LU et al.** *J. Immunol,* 1994, vol. 153, 1687-1696
- **D'ANDREA et al.** *J. Immunol,* 1995, vol. 155, 2306-2310
- **GUMPERZ et al.** *J. Exp. Med.,* 1995, vol. 181, 1133-1144
- **LANIER et al.** *J. Immunol,* 1994, vol. 153, 2417-2428
- **LANIER et al.** *Immunol Today,* 1996, vol. 17, 86-91
- **PHILLIPS et al.** *Immunity,* 1996, vol. 5, 163-172
- **RAULET et al.** *Cell,* 1995, vol. 82, 697-700
- **SODERSTROM et al.** *J. Immunol,* 1997, vol. 159, 1072-1075

- **YANG.** *Cancer,* 1995, vol. 76, 687-694
- **PHILLIPS.** *Journal of Clinical Oncology,* 1987, vol. 5, 1933-194
- **SAMBROOK et al.** Molecular Cloning, A Laboratory Manual. Cold Spring Harbor Laboratory Press, 1989

SEQUENCE LISTING

<110> THE BOARD OF TRUSTEES OF THE LELAND STANFORD JUNIOR UNIVERSITY

<120> COMPOSITIONS AND METHODS FOR PROTECTING ORGANS, TISSUE AND CELLS FROM IMMUNE SYSTEM-MEDIATED DAMAGE

<130> 12932-003WO1

<140> PCT/US99/19337
<141> 1999-08-24

<150> US 09/382,088
<151> 1999-08-24

<150> US 60/097,640
<151> 1998-08-24

<160> 29

<170> FastSEQ for Windows Version 4.0

<210> 1
<211> 9
<212> PRT
<213> Artificial Sequence

<220>

<223> consensus sequence

<221> VARIANT
<222> 2
<223> Xaa = Val, Leu, Ala, or Thr

<221> VARIANT
<222> 3
<223> Xaa = Leu or His

<221> VARIANT
<222> 4
<223> Xaa = Ser or Val

<221> VARIANT
<222> 6
<223> Xaa = Asp or Glu

<221> VARIANT
<222> 7
<223> Xaa = Gln, Lys, or Arg

<221> VARIANT
<222> (8)...(8)
<223> Xaa = Leu or Val

<400> 1

```
Ala Xaa Xaa Xaa Ala Xaa Xaa Xaa Arg
 1                   5
```

<210> 2
<211> 9
<212> PRT
<213> Artificial Sequence

<220>
<223> consensus sequence

<221> VARIANT
<222> 2
<223> Xaa = Val or Ala

<221> VARIANT
<222> 3
<223> Xaa = Leu or His

<221> VARIANT
<222> 4
<223> Xaa = Ser or Val

<400> 2

```
Ala Xaa Xaa Xaa Ala Glu Gln Leu Arg
 1                   5
```

```
<210> 3
<211> 9
<212> PRT
<213> Homo sapiens

<400> 3


                      Ala Val Leu Ser Ala Glu Gln Leu Arg
                       1               5


<210> 4
<211> 1254
<212> DNA
<213> Homo sapiens

<400> 4
```

```
ctataactcg tctcgcatct tgtcaccctt gagccggacc aggcgcccaa tgaatagcag      60
ggagccgctt tgggtgtccc gcaccaggaa gatgaagggg tggtcggcgt agaacagctt     120
ggggctgcgc agctcctcgc gcccgtagat gtcctggtca aaggggttgc catctgtgtc     180
caactcaaag gcggtggcgt ggaacacact ggccaggtac agatccttct tgccagacat     240
gcgtgataag tcggccttgt tcttgtcaat ggcctcagtc aggcccagcc cagccaggtg     300
tttctgcagg tcatgggtca cctccaccac acccttgggc aaggagatgg caacagcctt     360
cttctgcatc ttccccatcc agatcttcag ctgctctttg gttagcagct tttcaaggcg     420
ctcgagaggc tccacgtgat ggggcatgag gatgatgagg ctggagagct tgtgagccag     480
gggcatctcc accagctgca gcttctcctt ctcgtcgtcg tagtagttgt agaggcctgt     540
ccggtgcatc atcgtaacac ccacagtata ggaccgagtc accatgaagc cacggttgtc     600
caccatcttg tggtggaatt tctcatccca gtgtggcttg aagaacatgg cgttgactag     660
cagggcgccg tccgtgctct ccacgtcctt ggtgacctcg ggcagcttgc cgtcggtggt     720
ctgcgcggcc cactcgttga tggactgcag cgcgctgcgc ttgtccggga agttgatctt     780
ggagtgctcg cagttgtagt gctgcttgct gctcgccacg aagtcatcag cgaagctcac     840
tgagctgggt ccgtacagtc ggctgcccag cttccaggtc acgttgcgcg ccgtcgagtt     900
gctgagtgag cgcagcagct cacccaggcc ggcgtgcacc tcctcgtcgc gcagctgctc     960
ggcgctcagc actgccttgg cctgcgacgc cgtggtcgcc ttgccgccca gcgacacgag    1020
acccagcgac gaggccacca ccacgggtga caccaggatg ttctccactg cctggtcctt    1080
ggccattgcc tgatacaggc tgaaggccag gcctgtgctg ggctctgcca gtgtggtcgc    1140
```

```
cttggaactc agcttctccg cagtaccagg ggctgcggcc tctacaggtt tcttcacctc    1200
ggctgccagg gccacagcca ggaggcataa ggtgcccaga aggagagagc gcat          1254
```

```
<210> 5
<211> 1254
<212> DNA
<213> Homo sapiens

<220>
<221> CDS
<222> (1)...(1251)

<400> 5
```

EP 1 107 789 B1

```
atg cgc tct ctc ctt ctg ggc acc tta tgc ctc ctg gct gtg gcc ctg          48
Met Arg Ser Leu Leu Leu Gly Thr Leu Cys Leu Leu Ala Val Ala Leu
 1               5                  10                  15

gca gcc gag gtg aag aaa cct gta gag gcc gca gcc cct ggt act gcg          96
Ala Ala Glu Val Lys Lys Pro Val Glu Ala Ala Ala Pro Gly Thr Ala
             20                  25                  30

gag aag ctg agt tcc aag gcg acc aca ctg gca gag ccc agc aca ggc          144
Glu Lys Leu Ser Ser Lys Ala Thr Thr Leu Ala Glu Pro Ser Thr Gly
             35                  40                  45

ctg gcc ttc agc ctg tat cag gca atg gcc aag gac cag gca gtg gag          192
Leu Ala Phe Ser Leu Tyr Gln Ala Met Ala Lys Asp Gln Ala Val Glu
         50                  55                  60

aac atc ctg gtg tca ccc gtg gtg gtg gcc tcg tcg ctg ggt ctc gtg          240
Asn Ile Leu Val Ser Pro Val Val Val Ala Ser Ser Leu Gly Leu Val
 65                  70                  75                  80

tcg ctg ggc ggc aag gcg acc acg gcg tcg cag gcc aag gca gtg ctg          288
Ser Leu Gly Gly Lys Ala Thr Thr Ala Ser Gln Ala Lys Ala Val Leu
                 85                  90                  95

agc gcc gag cag ctg cgc gac gag gag gtg cac gcc ggc ctg ggt gag          336
Ser Ala Glu Gln Leu Arg Asp Glu Glu Val His Ala Gly Leu Gly Glu
             100                 105                 110

ctg ctg cgc tca ctc agc aac tcg acg gcg cgc aac gtg acc tgg aag          384
Leu Leu Arg Ser Leu Ser Asn Ser Thr Ala Arg Asn Val Thr Trp Lys
             115                 120                 125

ctg ggc agc cga ctg tac gga ccc agc tca gtg agc ttc gct gat gac          432
Leu Gly Ser Arg Leu Tyr Gly Pro Ser Ser Val Ser Phe Ala Asp Asp
         130                 135                 140

ttc gtg gcg agc agc aag cag cac tac aac tgc gag cac tcc aag atc          480
Phe Val Ala Ser Ser Lys Gln His Tyr Asn Cys Glu His Ser Lys Ile
145                 150                 155                 160

aac ttc ccg gac aag cgc agc gcg ctg cag tcc atc aac gag tgg gcc          528
Asn Phe Pro Asp Lys Arg Ser Ala Leu Gln Ser Ile Asn Glu Trp Ala
             165                 170                 175

gcg cag acc acc gac ggc aag ctg ccc gag gtc acc aag gac gtg gag          576
Ala Gln Thr Thr Asp Gly Lys Leu Pro Glu Val Thr Lys Asp Val Glu
```

```
                    180                      185                      190

      agc acg gac ggc gcc ctg cta gtc aac gcc atg ttc ttc aag cca cac        624
      Ser Thr Asp Gly Ala Leu Leu Val Asn Ala Met Phe Phe Lys Pro His
              195                 200                 205

      tgg gat gag aaa ttc cac cac aag atg gtg gac aac cgt ggc ttc atg        672
      Trp Asp Glu Lys Phe His His Lys Met Val Asp Asn Arg Gly Phe Met
              210                 215                 220

      gtg act cgg tcc tat act gtg ggt gtt acg atg atg cac cgg aca ggc        720
      Val Thr Arg Ser Tyr Thr Val Gly Val Thr Met Met His Arg Thr Gly
      225                 230                 235                 240

      ctc tac aac tac tac gac gac gag aag gag aag ctg cag ctg gtg gag        768
      Leu Tyr Asn Tyr Tyr Asp Asp Glu Lys Glu Lys Leu Gln Leu Val Glu
                          245                 250                 255

      atg ccc ctg gct cac aag ctc tcc agc ctc atc atc ctc atg ccc cat        816
      Met Pro Leu Ala His Lys Leu Ser Ser Leu Ile Ile Leu Met Pro His
                      260                 265                 270

      cac gtg gag cct ctc gag cgc ctt gaa aag ctg cta acc aaa gag cag        864
      His Val Glu Pro Leu Glu Arg Leu Glu Lys Leu Leu Thr Lys Glu Gln
              275                 280                 285

      ctg aag atc tgg atg ggg aag atg cag aag aag gct gtt gcc atc tcc        912
      Leu Lys Ile Trp Met Gly Lys Met Gln Lys Lys Ala Val Ala Ile Ser
              290                 295                 300

      ttg ccc aag ggt gtg gtg gag gtg acc cat gac ctg cag aaa cac ctg        960
      Leu Pro Lys Gly Val Val Glu Val Thr His Asp Leu Gln Lys His Leu
      305                 310                 315                 320

      gct ggg ctg ggc ctg act gag gcc att gac aag aac aag gcc gac tta       1008
      Ala Gly Leu Gly Leu Thr Glu Ala Ile Asp Lys Asn Lys Ala Asp Leu
                      325                 330                 335

      tca cgc atg tct ggc aag aag gat ctg tac ctg gcc agt gtg ttc cac       1056
      Ser Arg Met Ser Gly Lys Lys Asp Leu Tyr Leu Ala Ser Val Phe His
                      340                 345                 350

      gcc acc gcc ttt gag ttg gac aca gat ggc aac ccc ttt gac cag gac       1104
      Ala Thr Ala Phe Glu Leu Asp Thr Asp Gly Asn Pro Phe Asp Gln Asp
                      355                 360                 365

      atc tac ggg cgc gag gag ctg cgc agc ccc aag ctg ttc tac gcc gac       1152
      Ile Tyr Gly Arg Glu Glu Leu Arg Ser Pro Lys Leu Phe Tyr Ala Asp
              370                 375                 380

      cac ccc ttc atc ttc ctg gtg cgg gac acc caa agc ggc tcc ctg cta       1200
      His Pro Phe Ile Phe Leu Val Arg Asp Thr Gln Ser Gly Ser Leu Leu
      385                 390                 395                 400

      ttc att ggg cgc ctg gtc cgg ctc aag ggt gac aag atg cga gac gag       1248
      Phe Ile Gly Arg Leu Val Arg Leu Lys Gly Asp Lys Met Arg Asp Glu
                      405                 410                 415

      tta tag                                                               1254
```

Leu

<210> 6
<211> 417
<212> PRT
<213> Homo sapiens

<400> 6

```
Met Arg Ser Leu Leu Leu Gly Thr Leu Cys Leu Leu Ala Val Ala Leu
1               5               10                  15
Ala Ala Glu Val Lys Lys Pro Val Glu Ala Ala Ala Pro Gly Thr Ala
            20              25                  30
Glu Lys Leu Ser Ser Lys Ala Thr Thr Leu Ala Glu Pro Ser Thr Gly
        35              40                  45
Leu Ala Phe Ser Leu Tyr Gln Ala Met Ala Lys Asp Gln Ala Val Glu
        50              55                  60
Asn Ile Leu Val Ser Pro Val Val Val Ala Ser Ser Leu Gly Leu Val
65                  70                  75                  80
Ser Leu Gly Gly Lys Ala Thr Thr Ala Ser Gln Ala Lys Ala Val Leu
                85                  90                  95
Ser Ala Glu Gln Leu Arg Asp Glu Glu Val His Ala Gly Leu Gly Glu
            100             105                 110
Leu Leu Arg Ser Leu Ser Asn Ser Thr Ala Arg Asn Val Thr Trp Lys
            115             120                 125
Leu Gly Ser Arg Leu Tyr Gly Pro Ser Ser Val Ser Phe Ala Asp Asp
        130             135                 140
Phe Val Ala Ser Ser Lys Gln His Tyr Asn Cys Glu His Ser Lys Ile
145             150                 155                 160
Asn Phe Pro Asp Lys Arg Ser Ala Leu Gln Ser Ile Asn Glu Trp Ala
                165                 170                 175
Ala Gln Thr Thr Asp Gly Lys Leu Pro Glu Val Thr Lys Asp Val Glu
            180                 185                 190
Ser Thr Asp Gly Ala Leu Leu Val Asn Ala Met Phe Phe Lys Pro His
        195                 200                 205
Trp Asp Glu Lys Phe His His Lys Met Val Asp Asn Arg Gly Phe Met
    210                 215                 220
Val Thr Arg Ser Tyr Thr Val Gly Val Thr Met Met His Arg Thr Gly
225                 230         ---         235                 240
Leu Tyr Asn Tyr Tyr Asp Asp Glu Lys Glu Lys Leu Gln Leu Val Glu
                245                 250                 255
Met Pro Leu Ala His Lys Leu Ser Ser Leu Ile Ile Leu Met Pro His
            260                 265                 270
His Val Glu Pro Leu Glu Arg Leu Glu Lys Leu Leu Thr Lys Glu Gln
        275                 280                 285
Leu Lys Ile Trp Met Gly Lys Met Gln Lys Lys Ala Val Ala Ile Ser
        290                 295                 300
Leu Pro Lys Gly Val Val Glu Val Thr His Asp Leu Gln Lys His Leu
305                 310                 315                 320
Ala Gly Leu Gly Leu Thr Glu Ala Ile Asp Lys Asn Lys Ala Asp Leu
                325                 330                 335
Ser Arg Met Ser Gly Lys Lys Asp Leu Tyr Leu Ala Ser Val Phe His
            340                 345                 350
Ala Thr Ala Phe Glu Leu Asp Thr Asp Gly Asn Pro Phe Asp Gln Asp
        355                 360                 365
Ile Tyr Gly Arg Glu Glu Leu Arg Ser Pro Lys Leu Phe Tyr Ala Asp
        370                 375                 380
His Pro Phe Ile Phe Leu Val Arg Asp Thr Gln Ser Gly Ser Leu Leu
385                 390                 395                 400
Phe Ile Gly Arg Leu Val Arg Leu Lys Gly Asp Lys Met Arg Asp Glu
                405                 410                 415
Leu
```

<210> 7
<211> 9
<212> PRT

<213> Artificial Sequence

<220>
<223> synthetically generated peptide

<400> 7

Ala Ala His Val Ala Glu Gln Leu Arg
1               5

<210> 8
<211> 9
<212> PRT
<213> Rattus norvegicus

<400> 8

Ala Val Leu Ser Ala Glu Lys Leu Arg
1               5

<210> 9
<211> 9
<212> PRT
<213> Gallus gallus

<400> 9

Ala Val Leu Ser Ala Asp Lys Leu Asn
1               5

<210> 10
<211> 9
<212> PRT
<213> Homo sapiens

<400> 10

Ala Thr Leu Ser Ala Glu Arg Val Arg
1               5

<210> 11
<211> 9
<212> PRT
<213> Felis catus

<400> 11

Ala Thr Leu Ser Ala Glu Lys Val Arg
1               5

<210> 12
<211> 9
<212> PRT

<213> Bos taurus

<400> 12

                                    Ala Leu Leu Ser Ala Glu Lys Val Asn
                                     1                   5

<210> 13
<211> 9
<212> PRT
<213> Artificial Sequence

<220>
<223> consensus sequence

<221> VARIANT
<222> 6
<223> Xaa = Asp or Glu

<221> VARIANT
<222> 7
<223> Xaa = Lys or Gln

<400> 13

                                    Ala Val Leu Ser Ala Xaa Xaa Leu Arg
                                     1                   5

<210> 14
<211> 9
<212> PRT
<213> Artificial Sequence

<220>
<223> consensus sequence

<221> VARIANT
<222> 2
<223> Xaa = Val, Leu, or Thr

<221> VARIANT
<222> 7
<223> Xaa = Gln, Lys, or Arg

<221> VARIANT
<222> 8
<223> Xaa = Leu or Val

<400> 14

                                    Ala Xaa Leu Ser Ala Glu Xaa Xaa Arg
                                     1                   5

<210> 15
<211> 21

<212> DNA
<213> Artificial Sequence

<220>
<223> primer for PCR

<400> 15
acgtttggat ccaggtgaag a          21

<210> 16
<211> 12
<212> DNA
<213> Artificial Sequence

<220>
<223> primer for PCR

<400> 16
gtccttggcc at          12

<210> 17
<211> 27
<212> DNA
<213> Artificial Sequence

<220>
<223> primer for PCR

<400> 17
gcaatggcca aggaccaggc agtggag          27

<210> 18
<211> 27
<212> DNA
<213> Artificial Sequence

<220>
<223> primer for PCR

<400> 18
atctgaattc ctataactcg tctcgca          27

<210> 19
<211> 4
<212> PRT
<213> Homo sapiens

<400> 19

                                        Lys Asp Glu Leu
                                         1

<210> 20
<211> 4
<212> PRT
<213> Homo sapiens

<400> 20

                                       Arg Asp Glu Leu
                                        1

<210> 21
<211> 17
<212> PRT
<213> Homo sapiens

<400> 21

Met Arg Ser Leu Leu Leu Gly Thr Leu Cys Leu Leu Ala Val Ala Leu
 1                 5             10             15

Ala

<210> 22
<211> 4
<212> PRT
<213> Homo sapiens

<400> 22

                                         Arg Val Glu Leu
                                        1

<210> 23
<211> 27
<212> DNA
<213> Artificial Sequence

<220>
<223> primer for PCR

<400> 23
cggaattctg gccgaggtga agaaacc       27

<210> 24
<211> 27
<212> DNA
<213> Artificial Sequence

<220>
<223> primer for PCR

<400> 24
agttcccact gttctacgac ctagggc       27

<210> 25
<211> 28
<212> DNA
<213> Artificial Sequence

<220>
<223> primer for PCR

<400> 25
aactcaacct gtgtctagac ctatgggc          28

<210> 26
<211> 27
<212> DNA
<213> Artificial Sequence

<220>
<223> primer for PCR

<400> 26
acgcgctgct cctccacgac ctagggc          27

<210> 27
<211> 9
<212> PRT
<213> Artificial Sequence

<220>
<223> consensus sequence

<221> VARIANT
<222> 2, 4
<223> Xaa = Val or Ala

<221> VARIANT
<222> 3
<223> Xaa = Leu or His

<400> 27

```
                    Ala Xaa Xaa Xaa Ala Glu Gln Leu Arg
                     1                   5
```

<210> 28
<211> 9
<212> PRT
<213> Artificial Sequence

<220>
<223> consensus sequence

<221> VARIANT
<222> 2
<223> Xaa = Val, Leu, Ala, or Thr

<221> VARIANT
<222> 3
<223> Xaa = Leu or His

<221> VARIANT
<222> 4
<223> Xaa = Ala or Val

<221> VARIANT
<222> 6
<223> Xaa = Asp or Glu

<221> VARIANT
<222> 7
<223> Xaa = Lys, Gln, or Arg

<221> VARIANT
<222> (8)...(8)
<223> Xaa = Leu or Val

<400> 28

```
          Ala Xaa Xaa Xaa Ala Xaa Xaa Xaa Arg
               1                   5
```

<210> 29
<211> 9
<212> PRT
<213> Artificial Sequence

<220>
<223> consensus sequence

<221> VARIANT
<222> (1)...(9)
<223> Xaa = any amino acid

<400> 29

```
          Ala Xaa Xaa Xaa Ala Glu Gln Leu Arg
               1                   5
```